# EUROPEAN PATENT SPECIFICATION

(11) **EP 2 994 540 B1**
(45) Date of publication and mention of the grant of the patent: **08.12.2021**
(21) Application number: 14724142.6
(22) Date of filing: 08.05.2014
(51) Int. Cl.: C12Q 1/6886

(54) **CGB2 AND CGB1 GENES; DIAGNOSIS AND MONITORING OF CANCER**
CGB2- UND CGB1-GENE; DIAGNOSE UND ÜBERWACHUNG VON KREBS
GÈNES CGB2 ET CGB1; DIAGNOSTIC ET SUIVI DE CANCER

(30) Priority: 08.05.2013 GB 201308259
(43) Date of publication of application: 16.03.2016
(73) Proprietor: MAP IP HOLDING LIMITED, Ely, CB6 3FQ (GB)
(72) Inventor: ILES, Ray Kruse, Ely, Cambridgeshire CB6 3FQ (GB); BUTLER, Stephen Andrew, Ely, Cambridgeshire CB6 3FQ (GB)
(74) Representative: Dehns
(86) International application number: PCT/GB2014/051412
(87) International publication number: WO 2014/181115

(56) References cited:
- EP-A1- 2 161 038
- KOBROULY L ET AL: "HUMAN CHORIONIC GONADOTROPIN BETA SUBUNIT GENE EXPRESSION BY COMMON EPITHELIAL CANCERS-SPECIFIC SILENCING OF CGB1 & 2 GENES", TUMOR BIOLOGY, KARGER, BASEL, CH, vol. 31, no. Suppl.1, 1 August 2010 (2010-08-01), page S77, XP009179206, ISSN: 1010-4283
- K. RULL ET AL: "Fine-scale quantification of HCG beta gene transcription in human trophoblastic and non-malignant non-trophoblastic tissues", MOLECULAR HUMAN REPRODUCTION, vol. 14, no. 1, 1 January 2008 (2008-01-01), pages 23-31, XP055130224, ISSN: 1360-9947, DOI: 10.1093/molehr/gam082
- Y GIOVANGRANDI ET AL: "Analysis of the human CGB/LHB gene cluster in breast tumors by real-time quantitative RT-PCR assays", CANCER LETTERS, vol. 168, no. 1, 1 July 2001 (2001-07-01), pages 93-100, XP055130227, ISSN: 0304-3835, DOI: 10.1016/S0304-3835(01)00496-7
- K. RULL: "Expression of -subunit of HCG genes during normal and failed pregnancy", HUMAN REPRODUCTION, vol. 20, no. 12, 29 July 2005 (2005-07-29) , pages 3360-3368, XP055130234, ISSN: 0268-1161, DOI: 10.1093/humrep/dei261
- A. M. PARROTT ET AL: "Expression of Type II Chorionic Gonadotropin Genes Supports a Role in the Male Reproductive System", MOLECULAR AND CELLULAR BIOLOGY, vol. 31, no. 2, 15 November 2010 (2010-11-15), pages 287-299, XP055131664, ISSN: 0270-7306, DOI: 10.1128/MCB.00603-10
- HALLAST P ET AL: "The evolution and genomic landscape of CGB1 and CGB2 genes", MOLECULAR AND CELLULAR ENDOCRINOLOGY, ELSEVIER IRELAND LTD, IE, vol. 260-262, 2 January 2007 (2007-01-02), pages 2-11, XP026859643, ISSN: 0303-7207 [retrieved on 2006-12-13]
- ILES R K ET AL: "Does hCG or hCGbeta play a role in cancer cell biology?", MOLECULAR AND CELLULAR ENDOCRINOLOGY, ELSEVIER IRELAND LTD, IE, vol. 329, no. 1-2, 25 November 2010 (2010-11-25), pages 62-70, XP027326386, ISSN: 0303-7207 [retrieved on 2010-09-24]
- MARTA KUBICZAK ET AL: "Human Chorionic Gonadotropin Beta Subunit Genes CGB1 and CGB2 are Transcriptionally Active in Ovarian Cancer", INTERNATIONAL JOURNAL OF MOLECULAR SCIENCES, vol. 14, no. 6, 17 June 2013 (2013-06-17), pages 12650-12660, XP055130831, ISSN: 1661-6596, DOI: 10.3390/ijms140612650

## Description

### FIELD OF THE INVENTION

The present invention relates to methods for screening human tissue or fluid samples for changes in the expression of genes characteristic of poor prognosis in cancer such as bladder cancer. The methods have application for the screening ,diagnosis, or monitoring of other common epithelial cancers including those of the bladder, breast, cervix, colon, endometrium, kidney, lung (including small cell lung carcinoma - SCLC), nasal/pharynx, oro/facial, ovary, prostate, pancreas, vagina and vulva.

### BACKGROUND TO THE INVENTION

There are very few definitive tests for cancer and diagnosis is usually only confirmed with cytology. The sampling of cells only comes after presentation of symptoms and at this time it is common for the cancer to have advanced beyond the point of treatment efficacy. At this point cancers are often fatal. Current methods of cancer diagnostics include behavioural, genetic, biochemical, chemical and histological markers and are often highly specific for a particular type or subtype of cancer. Specific genes of human chorionic gonadotrophin (hCG) and its subunits have been suggested as markers of cancer before as have their genes (JM Bidart Patent US 6,194,154).

Detection of hCG is most commonly used in the detection and monitoring of gestational trophoblastic disease and germ cell tumours of the ovary and testis. The beta subunit of hCG (hCGβ) is used rarely although applications exist. hCG is a glycopeptide hormone produced by the syncytiotrophoblasts of the fetal placenta, and has a molecular weight of about 36 kilodaltons. It can be detected by immunoassay in the maternal serum and urine within days after fertilisation. The intact hCG molecule is a heterodimer comprising a specific β subunit non-covalently bound to an α subunit, which is common to other glycoproteins. hCGβ is expressed ectopically by approximately 32% of all epithelial cancers and is associated with poor prognosis, rapid metastasis and early mortality.

Because hCG is only usually found in pregnancy, any detectable levels of hCGβ can be indicative of cancer; but the extent of elevation can be highly variable and can be detected in both the serum and urine of cancer patients; as well as the cancerous tissue itself. hCG and hCGβ both degrade to a urinary product called human chorionic gonadotrophin beta core fragment (hCGβcf).

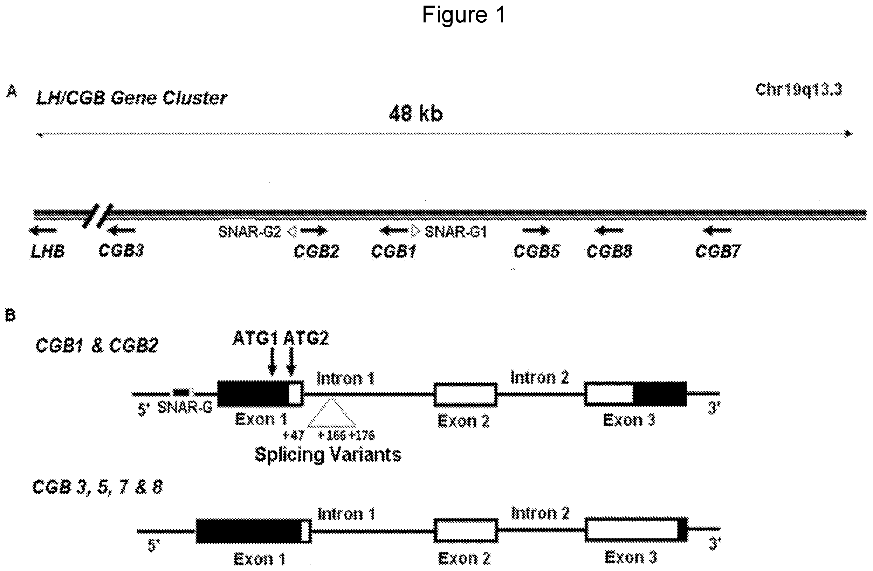

hCGβ is expressed from a region on chromosome 19 at 19q 13.32 by a cluster of 6 hCGβ gene paralogs originally designated CGB1, CGB2, CGB3, CGB5, CGB7 and CGB8 adjacent to the CGB4 gene encoding the hLHβ subunit. Later sequencing studies showed that genes CGB7 and CGB3 also have allelic variants, *CGB6* and *CGB9* respectively. CGB3 is now simply reported as CGB and was probably the first true CGB gene in the cluster arising adjacent to the LHβ gene (CGB4). See figure 1.

In pregnancy and cancer mRNA analysis has showed that all CG genes are active but the extent varies greatly. *CGB1* and *CGB2* have been regarded as pseudo genes since they were first described in 1982 because it appeared that they could not produce a mature hCGβ protein. The message arising from either gene CGB1 or CGB2 was noted to be shorter than those from the other genes as a result of alternative splicing and transcription results in three splice variants, only one of which represents a product that may translate into a functional hCGβ protein and this is considered unlikely. More recently, studies suggest CGB1 splice variants were up-regulated to some extent in the first trimester. Following the initial complete cloning of the LH-CGβ/ gene cluster other studies indicated that there were two groups of hCGβ producing genes - termed types I and II. Type I refers to the gene product of CGB7 which appears to lead to the synthesis of an hCGβ protein with an alanine residue at position 117 and type II refers to the gene products of *CGB*, CGB5 and CGB8 which all encode an aspartic acid residue at position 117. These studies formed the basis of JM Bidart US Patent No. 6,194,154. More recently other studies on breast, lung and renal carcinoma all indicate that CGB7 is expressed at a comparatively low level inconsistent with these initial claims.

The incorporation of CGB1 and CGB2 within the human genome is believed to have evolved in the cluster as a result of an insertion of a DNA fragment (736 bp for CGB1 and 724 bp for CGB2) that replaced the 52 bp sequence at the proximal end of the promoter and also the entire 5'-UTR of the ancestral hCGβ-subunit coding gene fragment, which is still present in *CGB*, *CGB5*, *CGB7* & *CGB8*. A one base pair frameshift in the ORF for exons 2 and 3 in genes *CGB1* and *CGB2* has been proposed to result in an optional earlier stop codon and shorter exon 3 in the transcripts. Therefore, unlike *CGB, CGB5,* CGB7 & CGB8 that each produces a single mRNA transcript; CGB1 and CGB2 potentially have multiple splice variants which bear little or no similarity to mature hCGβ. The variants include a predominant 230bp transcript coding a theoretical amino acid protein (which has never been isolated or characterised to date) and three other splice variants of CGB1 and/or CGB2 genes: +47, +166, +176 bp (Figure 1), that contain additional 47, 166 and 176bp DNA sequences taken from within intron 1. An alternative theoretical form of +47bp variant would result from the re-shift of the ORF to that of the classical hCGβ-coding transcripts and would therefore produce an additional short transcript (essentially another exon, which has been termed exon 1b) from the middle of intron 1. Alternative mRNAs +166bp and +176bp may hypothetically code for a shorter polypeptide of about 60 amino acids in length.

Expression of CGB1 and/or CGB2 (principally CGB2) specifically correlates with the secretion of cancer metastasis associated hCGβ protein (CMAhCGβ); whilst detection of CGB genes CGB3, *CGB5,* CGB7 and CGB8 mRNA does not correlate. Thus CGB1 and/or CGB2 mRNA detection is diagnostic of a metastatic and aggressive cancer phenotype.

It appears that CGB7 is always expressed at a lower level than genes CGB1 & CGB2 (which are always overlooked). CGB1 & CGB2 are expressed to a greater degree in cancer than *CGB*, *CGB5* and *CGB8* and form the basis of the present invention because they are responsible for the expression and secretion of an hCGβ protein which is associated with metastatic cancer.

L. Kobrouly et al. (Tumor Biology, vol. 31 (2010), page S77) disclose expression of the beta subunit of the hCG protein including the "pseudogenes" CGB1 and CGB2. Transcripts of CGB1 and CGB2 were detected in certain cell lines secreting hCGβ into the culture medium. Components suitable for detecting the expression of CGB1 and CGB2 were disclosed by K. Rull (Molecular Human Reproduction, vol. 14 (2008), pages 23-31), Y. Giovangrandi et al. (Cancer Letters, vol. 168 (2001), pages 93-100), K. Rull (Human Reproduction, vol. 20 (2005), pages 3360-3368), and A.M. Parrott et al. (Molecular and Cellular Biology, vol. 31 (2010), pages 287-299).

### STATEMENTS OF THE INVENTION

The present invention is defined by the appended claims.

According to the present invention, there is provided a method for investigating a cancerous condition, the method comprising demonstrating the expression of CGB1 and/or CGB2 genes/pseudogenes or a product of at least one of these genes/pseudogenes. Specifically the method detects and/or measures the expression of the 230bp splice variant of the CGB2 and/or CGB1 gene or a product of at least one of these genes. Preferably the product is the protein CMAhCGβ.

In another aspect the invention provides a kit for detecting the expression of CGB1 and/or CGB2 genes/pseudogenes or a product of at least one of these genes/pseudogenes

The invention also provides the expression product of CGB2 or CGB1, preferably the 230bp splice variant. Preferably the expression product is a nucleic acid sequence, a protein or peptide. Preferably the product is the protein CMAhCGβ.

The expression product of CGB2 and/or CGB1 can be used in the methods of the invention. Thus in a further aspect the invention provides an expression product of CGB2 and/or CGB1 for use in the investigation of a cancerous condition, preferably an epithelial cancer.

In another aspect the invention provides a composition comprising an agent capable of decreasing the level of secreted hCGβ for use in the treatment of a cancerous condition, preferably an epithelial cancer. Preferably the epithelial cancer has been found to secrete hCGβ. The composition can comprise agents which prevent the expression of hCGβ, for example siRNA molecules. Alternatively the composition may comprise agents which bind to the secreted hCGβ such as antibodies.

The disclosure also relates to a method of treating a cancerous condition, preferably an epithelial cancer, said method comprising administering to a subject in need thereof, an effective amount of an agent capable of decreasing the level of secreted hCGβ.

The invention also provides a composition comprising an agent capable of decreasing the level of secreted hCGβ.

### DETAILED DESCRIPTION OF THE INVENTION

CGB1 *and CGB2* are referred to herein as genes rather than pseudogenes because, although they have hitherto been regarded as pseudogenes, it has been shown in the present application that these genes are expressed.

According to the present invention, there is provided a method for investigating a cancerous condition, the method comprising demonstrating the expression of CGB2 and/or CGB1 genes/pseudogenes or a product of at least one of these genes/pseudogenes. Specifically the method detects and/or measures the expression of the 230bp splice variant of the CGB2 and/or CGB1 gene. Preferably, the expression is detected and/or measured by determining the presence and/or amount of and expression product such as mRNA, cDNA, a peptide or a protein. The presence of an expression product of *CGB2* and/or *CGB1* in a sample obtained from a subject is indicative of the presence of a CMAhCGβ secreting cancerous condition. In particular is the level of the expression product of *CGB2* and/or CGB1 is found to be in excess of the level of the expression product of the other CG genes, CGB3, *CGB5,* CGB7 and CGB8 , particularly 2 fold or more in excess then this is indicative of a CMAhCGβ secreting cancerous condition.

As used herein, "investigating" includes one or more methods of detecting, monitoring, screening, diagnosing and prognosing the cancerous condition. The presence of an expression product of the CGB1 and/or CGB2 genes is indicative of a cancer which secretes hCGβ. These cancers are aggressive forms of cancer i.e. metastatic and invasive, and generally have a poor prognosis for survival. Thus detection of an expression product of the CGB1 and/or CGB2 genes can diagnose the presence of a cancerous condition, and can be used in methods of screening subjects to identify those which have the cancerous condition. In addition it can give an indication of prognosis. Furthermore the methods of the invention can be used to monitor the progress of a cancerous condition. For example the methods can be used to identify whether a treatment regimen is effective, as a reduction in the level of the expression product may be indicative of a reduction the number of cancerous cells present.

The expression of the CGB2 and/or CGB1 genes can be demonstrated by detecting an expression product of the CGB2 and/or CGB1 genes. The "expression product" can be a nucleic acid molecule such as mRNA, or the amino acid sequence translated from the nucleic acid sequence such as a peptide, pre-protein or protein.

It has been found by the present inventors that the 230 bp splice variant of CGB2 and/or CGB1 (also referred to herein as CGβ2θ) is expressed. In this splice variant the entire sequence of intron 1 is removed, as shown in Figure 1. Thus 230bp transcript which is produced lacks intron 1, and Exon 1 abuts Exon 2. This is in contrast to the other theoretical splice variants where in some of intron 1 remains between Exons 1 and 2 (see Figure 1). These differences can be used to detect the presence of the 230bp transcript. The sequence of the 230 bp mRNA, CGβ2θ, is shown in SEQ. ID NO:1. The 230bp transcript encodes an amino acid sequence if translation is initiated at the second consensus ATG

(SEQ. ID NO:2). The pre-protein produced contains a sequence which is different to that of the pre-protein produced by the expression of the other CGB genes, *CGB3*, *CGB5*, *CGB7* and *CGB8*, as shown in Table 2. The difference in the pre-protein sequences can be used to detect the expression of the 230bp transcript. The pre-protein is processed to produce the mature protein, CMAhCGβ, as shown in Table 2

As used herein, reference to expression and expression products should be understood to refer to both the natural mRNA transcription products, and cDNA derived therefrom, as well as the natural translation products, such as the pre-protein and protein.

In a preferred embodiment the expression product detected is a nucleic acid, preferably mRNA. The presence of the nucleic acid can be detected by using methods based on the difference in the size and/or sequence between the 230bp transcript and other theoretical transcripts of CGB2 and/or CGB1 genes and the transcripts from the other CGB genes i.e. *CGB3*, *CGB5, CGB7* and *CGB8*. Such methods are known in the art. These methods include Reverse Transcriptase PCR (RT PCR), quantative PCR (QPCR), hybridisation techniques such as blotting following gel electrophoresis.or Capiliiry electrophoreseis and any nucleic acid sequencing methodologies.

In a preferred embodiment the method utilises primers which hybridise to the mRNA transcription product, and then the sequence may be amplified, using techniques such as PCR including reverse transcriptase PCR (rtPCR) and quantative (qPCR). Preferably the forward primer hybridises to a sequence within Exon 1. Preferably the forward primer is 5'-CGTCCAACACCCCTCACTCC-3' (SEQ ID No: 4.). Alternatively the forward primer hybridises to a sequence at the Exon1/Exon2 boundary, as this will specifically detect the 230bp transcript. The Exon1/Exon2 boundary occurs in the region between bases 110-115 of SEQ. ID NO: 1. The reverse primer can hybridise to any sequence after the initiation site in Exon 2. Preferably the reverse primer is 5'- 5 GGCAGCCCTCCTTCTCCAC-3' (SEQ ID. NO:5). Methods of selecting suitable primer sequences are known to the person skilled in the art. In one preferred method of the invention, use is made of one or both of the primers: Forward 5'-CGTCCAACACCCCTCACTCC-3' (SEQ ID No: 4.) and Reverse 5'-GGCAGCCCTCCTTCTCCAC-3' (SEQ ID. NO:5) or a variation thereof. A variation includes a similar primer having appropriate functionality, for instance, one with a single base pair substitution or deletion or one which would lead to the amplification of the 230bp transcript or part thereof.

Optionally following amplification, the presence of the 230bp transcript can be determined by separating the amplification products according to size, for example by using gel electrophoresis. Alternatively the presence of the amplified product can be determined using a probe which hybridises to the 230bp transcript. Preferably the probe selectively hybridises to the 230bp transcript i.e. it does not hybridise to the other splice variant transcripts of the CGB2 and/or CGB1 genes or the other CGB genes, CGB3, CGB5, CGB7 and CGB8.

Alternatively the presence of the 230bp mRNA transcription product can be detected using a probe which hybridises to the 230bp splice variant transcript. Preferably the probe selectively hybridises to the 230bp splice variant transcript i.e. it does not hybridise to the other splice variant transcripts of the CGB2 and/or CGB1 genes or the other CGB genes, CGB3, CGB5, CGB7 and CGB8. For example the probe may hybridise with a sequence at the Exon1/Exon2 boundary, as this will specifically detect the 230bp transcript. The Exon1/Exon2 boundary occurs in the region between bases 110-115 of SEQ. ID NO: 1. The hybridisation of the CGB2 and/ or CGB1 gene product can be detected using methods known in the art. For example by labelling the probe, or using the probe to initiate a sequencing reaction. Labelled nucleotides may be used to indicate that a sequence has been generated, and thus that hybridisation has occurred.

The probe may form part of an array. The term "array" as used herein relates to a spatially defined arrangement of one or more nucleotides in a pattern on a surface. The array can consist of individual nucleotides present at at least 96, 384, 536, 10,000, 48,000 or 192,000, 786, 000, 60 million discrete locations on a surface. The array is preferably formed on a chip. The array may be present within a microfluidic conduit. The arrays may also be on the bottom of microtitre plate or on flat bottomed microfuge tubes. These preferably have a bottom composed of high optical quality material.

The array can be a random array wherein the nucleotides are attached to the surface randomly. Alternatively the arrays can be spatially addressed. The nucleotides can be arranged in a grid pattern, with regular spacing between each nucleotide. The nucleotides can be located in a "spot" along with a plurality of other nucleotides of the same sequence. Alternatively the arrays can comprise DNA colonies.

Also the arrays can be composed of tandem copies of the same sequence within a single polymer as can be created by Rolling Circle Amplification (RCA) (Smirnov *et al*)*.*

The polynucleotides can be attached either directly or indirectly to the surface. For example an enzyme, such as a ligase or polymerase, ustilised in the process can be attached to a solid surface. The enzyme then binds the target polynucleotide, thus anchoring it to the solid surface.

Alternatively the polynucleotides can be captured by oligonucleotides which are attached to the surface. The capture can be by hybridisation of a single stranded oligonucleotide to a single stranded target or a single stranded region of a double stranded target. Alternatively, the polynucleotide or the surface immobilised capture probe, may comprise a sticky end or both may have a sticky end. The template and synthesized strand can be permanently linked to the surface by a ligation reaction. Alternatively the permanent fixing can be mediated by including a Psoralen moiety opposite a thymine residue and cross-linking with UV light.

Molecules can be attached to a solid surface by a number of methods that are well known to the person skilled in the art (such as those described by Fodor *et al* (1991), Hegner *et al* (1993), or Finzi and Gelles (1995). Suitable methods of using nucleotides to form an array, and attaching nucleotides to an array are described in WO02/061126 and WO01/57248.

During array synthesis or the preparation of oligonucelotides to make the array UniCap Phosphoramidite (Glen Research) can be used for efficient capping in oligonucelotide synthesis. This will prevent undesired n-1mers (truncated oligomers) from participating in subsequent sequencing by synthesis reactions.

The surface is preferably glass, silica or a polymer such as PMDS or a Fluoropolymer. The substrate is preferably a glass slide, coverslip, silicon wafer, microfabricated chip or multi-well plate, such as a flat bottomed optical grade 96 well plate. The polynucleotides may be attached to material that coats the surface. For example aminosilane coated surfaces supplied by Corning Inc (USA) or Asper Biotech (Estonia) can be used. The surface may be coated with a gel material including agarose, polyacrylamids or a sol-gel. The polynucleotides may be attached to beads, particles, or structures such as nanobars or nanorods which may contribute to the generation or modulation of a FRET signal. The surface may be metalized with for example silver or gold particles to enhance a fluorescent or a raman signal (Malicka 2003; Kneipp 1999).

In addition, the surface or particles thereon may carry charge or be electrically biased or may be heated in order to control the sequencing process (Hamad-Schifferli, 2002). A charged surface is particularly useful to prevent non-specific interactions of nucleotides on the surface. Appropriate surface coatings include Polyethylamine as described by Braslavsky, 2003 and the DNA-bind slide available from VBC Genomics (Austria). An electric field generated at the surface is a useful way for controlling the attraction and repulsion of nucleotides at the surface (Asanov 1998; Sosnowski 1997)

Compared to the degree of parallelism currently available (96 Sanger sequencing reactions within individual capillaries on a state-of-the-art DNA sequencer) a whole wafer high-density oligonucleotide array has the capacity to analyse 60 million reactions (e.g. see www.perlegen.com website).

Until recently, the high cost of making individual photolithography masks meant that methods for making high-density oligonucleotide arrays were only available for mass production of arrays and were not accessible for the individual design of single arrays. However, the application of a Texas Instruments' digital micromirror device (DMD) to array synthesis has made it much more straightforward and cheaper to specify individual arrays. The DMD is a chip comprising an array of 786,000 micromechanical aluminum mirrors, where each mirror is individually addressable. Using these tiny aluminum mirrors to shine light in specific patterns, coupled with the photo deposition chemistry, arrays of oligonucleotide probes are produced. Several companies and laboratories have implemented this technology, notably Xeotron and Nimblegen. A fully integrated benchtop device for making, hybridising to and analysing high-density arrays can streamline an entire miroarray experiment to within one day, (e.g Geniom one; Baum *et al*). The Geniom one uses the DMD to create an array by the spatially-selective deprotection of photolabile protecting groups on DNA chains growing on a surface. Each new array design can simply and rapidly be specified by software and there is no need to make photolithography masks. Arrays can be made such that the sequencing can be initiated either with an array of oligonucleotides directed to specific regions in the genome or with an array of n-mers (Gunderson *et al,* 1998) which will initiate the process at any position which seeds hybridisation. Presently, Geniom one is configured to synthesize 48,000 oligonucleotides. However, it is possible to synthesize at least 192,000 sequences on one chip in one synthesis run. All the synthesis, hybridisation and washing steps can be undertaken within the microfluidic channels of the chip provided by the manufacturer. The benefit of this system is that it can rapidly iterate array synthesis based on information that is obtained.

The primers and/or probe must have a sufficient level of identity with the CBG2 and/or CGB1 gene transcription product, such as the 230bp transcription product so that it can hybridize with the transcription product under suitable conditions. One single-stranded nucleic acid is said to hybridize to another if a duplex forms between them. This occurs when one nucleic acid contains a sequence that is the reverse or complement of the other (this same arrangement gives rise to the natural interaction between the sense and antisense strands of DNA in the genome and underlies the configuration of the double helix). Complete complementarity between the hybridizing regions is not required in order for a duplex to form; it is only necessary that the number of paired bases is sufficient to maintain the duplex under the hybridization conditions used. Suitable hybridization conditions are well known to the person skilled in the art. For example 0.2×SSC/0.1% SDS at 42° C. (for conditions of moderate stringency); and 0.1×SSC at 68° C. (for conditions of high stringency). Washing can be carried out using only one of the conditions given, or each of the conditions can be used (for example, washing for 10-15 minutes each in the order listed above). Any or all of the washes can be repeated. Optimal conditions will vary and can be determined empirically by the skilled person.

Alternatively the expression product detected in the methods of the invention is the protein or pre-protein. The pre-protein is the amino acid sequence that is produced upon translation of the 230bp transcript. The pre-protein is processed and cleaved to form the mature hCGβ which is secreted. The pre-protein contains a sequence which is not present in the mature protein. This sequence is different to that found in the pre-protein produced from the other CGB genes, CGB3, *CGB5, CGB7* and CGB8 and theoretically produced from the other splice variant transcripts of the CGB2 and/or CGB1 genes, as shown in Table 2.

Methods of detecting the pre-protein can be based on the difference in size and/or sequence between the pre-proteins produced from the 230bp transcript and those produced from the other CGB genes, CGB3, CGB5, *CGB7* and CGB8 and the theoretically produced from the other splice variant transcripts of the CGB2 and/or CGB1 genes. Such methods are known to the person skilled in the art and include methods utilising antibodies or lectins which bind specifically to pre-protein or protein translated from the CG genes. Preferably the antibodies or lectins bind specifically to the amino acid sequences present in the pre-protein but which are absent in the mature protein. Preferably the antibodies or lectins are specific to the pre-protein translated from the 230bp splice variant transcript, such as the protein of SEQ ID No.2 and/ or SEQ Id No. 16, or CMAhCGβ.

The term "antibody" as used herein refers to immunoglobulin molecules and immunologically active portions of immunoglobulin molecules, i.e., molecules that contain a binding site that specifically binds pre-protein or protein translated from the CG genes, whether natural or partly or wholly synthetic. The term also covers any polypeptide or protein having a binding domain which is, or is homologous to, an antibody binding domain. These can be derived from natural sources, or they may be partly or wholly synthetically produced. Examples of antibodies are the immunoglobulin isotypes (e.g., IgG, IgE, IgM, IgD and IgA) and their isotypic subclasses; fragments which comprise an antigen binding domain such as Fab, scFv, Fv, dAb, Fd; and diabodies. Antibodies may be polyclonal or monoclonal.

It is possible to take monoclonal and other antibodies and use techniques of recombinant DNA technology to produce other antibodies or chimeric molecules which retain the specificity of the original antibody. Such techniques may involve introducing DNA encoding the immunoglobulin variable region, or the complementary determining regions (CDRs), of an antibody to the constant regions, or constant regions plus framework regions, of a different immunoglobulin. See, for instance, EP-A-184187, GB 2188638A or EP-A-239400.

As antibodies can be made or modified in a number of ways, the term antibody should be construed as covering any specific binding member or substance having a binding domain with the required specificity. Thus, this term covers antibody fragments, derivatives, functional equivalents and homologues of antibodies, humanised antibodies, including any polypeptide comprising an immunoglobulin binding domain, whether natural or wholly or partially synthetic. Chimeric molecules comprising an immunoglobulin binding domain, or equivalent, fused to another polypeptide are therefore included. Cloning and expression of chimeric antibodies are described in EP-A-0120694 and EP-A-0125023. A humanised antibody may be a modified antibody having the variable regions of a non-human, e.g. murine, antibody and the constant region of a human antibody. Methods for making humanised antibodies are described in, for example, US Patent No. 5225539.

It has been shown that fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment (Ward, E.S. et al., Nature 341:544-546 (1989)) which consists of a VH domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al., Science 242:423-426 (1988); Huston et al., PNAS USA 85:5879-5883 (1988)); (viii) bispecific single chain Fv dimers (PCT/US92/09965) and (ix) "diabodies", multivalent or multispecific fragments constructed by gene fusion (WO94/13804; P. Hollinger et al., Proc. Natl. Acad. Sci. USA 90: 6444-6448 (1993)).

Diabodies are multimers of polypeptides, each polypeptide comprising a first domain comprising a binding region of an immunoglobulin light chain and a second domain comprising a binding region of an immunoglobulin heavy chain, the two domains being linked (e.g. by a peptide linker) but unable to associate with each other to form an antigen binding site: antigen binding sites are formed by the association of the first domain of one polypeptide within the multimer with the second domain of another polypeptide within the multimer (WO94/13804).

An "antigen binding domain" is the part of an antibody which comprises the area which specifically binds to part or all of the pre-protein or protein translated from the CG gene products. An antibody may only bind to a particular part of the pre-protein or protein translated from the CG gene products, which part is termed an epitope. An antigen binding domain may be provided by one or more antibody variable domains. An antigen binding domain may comprise an antibody light chain variable region (VL) and an antibody heavy chain variable region (VH).

"Specific" is generally used to refer to the situation in which the binding moiety will not show any significant binding to molecules other than its target(s), and, e.g., has less than about 30%, preferably 20%, 10%, 1%, 0.5%, 0.3%, 0.2% or 0.1% cross-reactivity with any other molecule.

Preferably, the cancerous condition is an epithelial cancer. "Epithelial cancers" as used herein include cancers of the bladder, breast, cervix, colon, endometrium, kidney, lung (including small cell lung carcinoma - SCLC), nasal/pharynx, oro/facial, ovary, prostate, pancreas, vagina and vulva. The method of the invention is particularly suitable for use in connection with an aggressive form of a cancerous condition. As used herein an "aggressive" cancerous condition is one which is metastatic and/or invasive.

The methods of the invention can be carried out on a sample obtained from a subject. The sample can be a blood, urine, cerebrospinal fluid, sputum, bronchial lavage, and saliva or tissue sample. Tissue samples include biopsy material, exfoliated cells, circulating tumour cells, brush biopsies, and cells obtained by swabbing e.g. cheek epithelial cells, and brush biopsy such as cervical smear samples. Biopsy material includes any cells obtained by any biopsy technique including brush biopsies, excisional biopsies, incisional biopsies, needle aspiration or core biopsies. The sample may also comprise circulating tumour cells and/or stem cells isolated from the blood of a subject. The methods of the invention can be used to determine the metastatic potential of circulating tumour cells and circulating stem cells found in the blood.

The cells may be isolated or enriched from the sample prior to detecting and/or measuring the *CGB2* and/or *CGB1* expression product, such as the product of the 230bp splice variant transcript. For example fluid samples can be centrifuged to obtain a pellet of cells; circulating tumour cells may be isolated from a blood sample. The cells may then be lysed in order to detect the expression product of the CGB2 and/or CGB1 expression product, such as the product of the 230bp splice variant transcript.

The methods of the invention may further comprise detecting and/or measuring the expression of any one or more of the genes CGB3 *CGB5, CGB7,* and/or CGB8. Preferably the expression of all of the genes CGB3 CGB5, *CGB7,* and/or CGB8 is detected and/or measured. Preferably where the expression level of the 230bp splice variant exceeds the level of expression from CGB3 CGB5, *CGB7,* and/or CGB8, this is indicative of the presence of an hCGβ secreting cancerous condition, such as a CMAhCGβ secreting cancerous condition. Preferably the expression level of the 230bp splice variant exceeds the level of expression from CGB3 CGB5, CGB7, and/or CGB8 by a factor of 2, 3, 5, 10 or more. Most preferably the presence of an hCGβ secreting cancerous condition, (such as a CMAhCGβ secreting cancerous condition) is indicated when the expression level of the 230bp splice variant exceeds the level of expression from CGB3 CGB5, CGB7, and/or *CGB8* by a factor of 2.

Thus method of the invention may further comprise using methods comprising nucleic acid hybridisation for the detection of the transcription product of the CGB genes, CGB3 CGB5, CGB7, and/or *CGB8*.

In a preferred embodiment the method utilises primers which hybridise to the mRNA transcription product, and then the sequence may be amplified, using techniques such as PCR including reverse transcriptase PCR (rtPCR) and quantative (qPCR). Preferably the forward primer hybridises to a sequence spanning exon1 to exon 2 boundary. Preferably the forward primer is 5'- CAGCACCTTTCTCGGGTCAC-3' (SEQ ID No: 6.) The reverse primer can hybridise to any sequence after the initiation site in Exon 2. Preferably the reverse primer is 5'- CAGGGAGTAGGGTGTAGGAAGG-3' (SEQ ID. NO:7). Or any primers defining a sequence of the 230bp splice variant 5' - CGTCCAACACCCCTCACTCCCTGTCTCACTCCCCCACGGAGACTCAATTTACTTTCCA TGTCCACATCCCCAGTGCTTGCGGAAGATATCCCGCTAAGAGAGAGACATGTCAAAG GGGCTGCTGCTGTTGCTGCTGCTGAGCATGGGCGGGACATGGGCATCCAAGGAGCC GCTTCGGCCACGGTGCCGCCCCATCAATGCCACCCTGGCTGTGGAGAAGGAGGGCT GCC - 3' (SEQ ID NO:1). Methods of selecting suitable primer sequences are known to the person skilled in the art. In one preferred method of the invention, use is made of one or both of the primers: Forward (5'- CAGCACCTTTCTCGGGTCAC-3') (SEQ ID No:6.) and Reverse (5'- CAGGGAGTAGGGTGTAGGAAGG-3') (SEQ ID No: 7) to detect expression of CGB3 CGB5,CGB7, and/or CGB8, or a variation thereof, in order to differentiate CGB gene expression. Again, a variation includes a similar primer having appropriate functionality, for instance, one with a single base pair substitution or deletion.

Optionally following amplification, the presence of the transcription product of the CGB genes, CGB3 *CGB5,* CGB7, and/or CGB8 can be determined by separating the amplification products according to size, for example by using gel electrophoresis.

Alternatively the presence of the amplified transcription product of the CGB genes, CGB3 CGB5, CGB7, and/or CGB8 can be determined using a probe which hybridises to the transcription product of the CGB genes, *CGB3 CGB5, CGB7,* and/or *CGB8*. . Preferably the probe selectively hybridises to transcription product of the CGB genes, CGB3 *CGB5,* CGB7, and/or CGB8 i.e. it does not hybridise to the 230bp transcript or other splice variant transcripts of the CGB2 and/or CGB1 genes.

Alternatively the presence of the mRNA transcription product of the CGB genes, CGB3 CGB5, CGB7, and/or CGB8 can be detected using a probe which hybridises to the transcription product of the CGB genes, CGB3 CGB5, CGB7, and/or *CGB8*. Preferably the probe selectively hybidises to the transcription product of the CGB genes, CGB3 CGB5, CGB7, and/or CGB8 i.e. it does not hybridise to the 230bp transcript or other splice variant transcripts of the CGB2 and/or CGB1 genes.

The primers and/or probe must have a sufficient level of identity with the transcription product of the CGB genes, CGB3 *CGB5,* CGB7, and/or CGB8 so that it can hybridize with the transcription product of the CGB genes, CGB3 *CGB5,* CGB7, and/or CGB8 under suitable conditions. One single-stranded nucleic acid is said to hybridize to another if a duplex forms between them. This occurs when one nucleic acid contains a sequence that is the reverse or complement of the other (this same arrangement gives rise to the natural interaction between the sense and antisense strands of DNA in the genome and underlies the configuration of the double helix). Complete complementarity between the hybridizing regions is not required in order for a duplex to form; it is only necessary that the number of paired bases is sufficient to maintain the duplex under the hybridization conditions used. Suitable hybridization conditions are well known to the person skilled in the art. For example 0.2×SSC/0.1% SDS at 42° C. (for conditions of moderate stringency); and 0.1×SSC at 68° C. (for conditions of high stringency). Washing can be carried out using only one of the conditions given, or each of the conditions can be used (for example, washing for 10-15 minutes each in the order listed above). Any or all of the washes can be repeated. Optimal conditions will vary and can be determined empirically by the skilled person.

Alternatively the method of the invention may further comprise detecting and/or measuring the expression of CGB3 CGB5, *CGB7,* and/or CGB8, by detecting and/or measuring the peptide, protein or pre-protein. The pre-protein is the amino acid sequence that is produced when the mRNA is translated. The pre-protein is processed and cleaved to form the mature hCGβ which is secreted. The pre-protein contains a sequence which is not present in the mature protein, as shown in Table 2. The pre-protein comprises SEQ ID NO:2 or SEQ ID No. 15. This sequence is different to that found in the pre-protein produced from the 230bp transcript and theoretically produced from the other splice variant transcripts of the *CGB2* and/or CGB1 genes, as shown in Table 2.

Methods of detecting the pre-protein can be based on the difference in size and/or sequence between the pre-proteins produced from the CGB genes, CGB3, CGB5, CGB7 and CGB8 and those produced from the 230bp transcript and the theoretically produced proteins from the other splice variant transcripts of the CGB2 and/or CGB1 genes. Such methods are known to the person skilled in the art and include methods utlising antibodies which bind specifically to pre-protein or protein translated from the CG genes (eg by western blotting). Preferably the antibodies bind specifically to the amino acid sequences present in the pre-protein but which are absent in the mature protein. Prefereably the antibodies are specific to the pre-protein translated from the CGB genes, *CGB3*, *CGB5*, *CGB7* and *CGB8* transcripts.

The present invention further provides an expression product of *CGB1* or *CGB2*. Preferably the expression product is the 230bp splice variant transcript. Preferably, the expression product is a mRNA transcript. The sequence of the 230 bp mRNA, CGβ2θ, is shown in SEQ. ID NO:1 In a preferred embodiment the expression product is nucleic acid which comprises
(a) A nucleic acid of SEQ. ID NO:1;
(b) A nucleic acid sequence encoding the polypeptide sequence of SEQ. ID No:2 or SEQ ID NO:15;
(c) A sequence substantially identical to the sequence of (a) or (b); or a homologue or variant thereof;
(d) A sequence complementary or hybridises to a sequence of any one of (a) to (c).

Preferably, the expression product is an amino acid sequence. The 230bp transcript encodes an amino acid sequence, as shown in SEQ. ID NO: 2. In a preferred embodiment the expression product is a polypeptide comprising SEQ. ID NO: 2 or SEQ ID NO:15 or a substantially identical sequence thereof, or a homologue or derivative thereof. Preferably the expression product is an amino acid with a N-Terminal sequence of SEQ ID No.2.

Preferably the amino acid comprises or consists of the sequence of SEQ ID No: 15

The skilled person will appreciate that homologues or derivatives of the proteins or polypeptides of the invention will also find use in the context of the present invention, i.e. as antigenic/immunogenic material. Thus, for instance proteins or polypeptides which include one or more additions, deletions, substitutions or the like are encompassed by the present invention. In addition, it may be possible to replace one amino acid with another of similar "type". For instance replacing one hydrophobic amino acid with another.

One can use a program such as the CLUSTAL^{™} program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of identity analysis are contemplated in the present invention.

In the case of homologues and derivatives, the degree of identity with a protein or polypeptide as described herein is less important than that the homologue or derivative should retain the antigenicity or immunogenicity of the original protein or polypeptide. However, suitably, homologues or derivatives having at least 60% similarity (as discussed above) with the proteins or polypeptides described herein are provided. Preferably, homologues or derivatives having at least 70% similarity, more preferably at least 80% similarity are provided. Most preferably, homologues or derivatives having at least 90% or even 95% similarity are provided.

In an alternative approach, the homologues or derivatives could be fusion proteins, incorporating moieties which render purification easier, for example by effectively tagging the desired protein or polypeptide. It may be necessary to remove the "tag" or it may be the case that the fusion protein itself retains sufficient antigenicity to be useful.

"Substantially identical" as used herein refers to a nucleic acid or amino acid sequence having at least 60% identity. The sequences of the invention may have at least 60%,70%, 80%, 90% , 95%, 97.5% or 99% identity.

The percent identity of two amino acid sequences or of two nucleic acid sequences is determined by aligning the sequences for optimal comparison purposes (*e*.*g*., gaps can be introduced in the first sequence for best alignment with the sequence) and comparing the amino acid residues or nucleotides at corresponding positions. The "best alignment" is an alignment of two sequences which results in the highest percent identity. The percent identity is determined by the number of identical amino acid residues or nucleotides in the sequences being compared (*i*.*e*., % identity = number of identical positions/total number of positions x 100).

The determination of percent identity between two sequences can be accomplished using a mathematical algorithm known to those of skill in the art. An example of a mathematical algorithm for comparing two sequences is the algorithm of Karlin and Altschul (1990) Proc. Natl. Acad. Sci. USA 87:2264-2268, modified as in Karlin and Altschul (1993) Proc. Natl. Acad. Sci. USA 90:5873-5877. The NBLAST and XBLAST programs of Altschul, et al. (1990) J. Mol. Biol. 215:403-410 have incorporated such an algorithm. BLAST nucleotide searches can be performed with the NBLAST program, score = 100, wordlength = 12 to obtain nucleotide sequences homologous to nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilised as described in Altschul et al. (1997) Nucleic Acids Res. 25:3389-3402. Alternatively, PSI-Blast can be used to perform an iterated search which detects distant relationships between molecules (*Id.*)*.* When utilising BLAST, Gapped BLAST, and PSI-Blast programs, the default parameters of the respective programs (e.g., XBLAST and NBLAST) can be used. *See* http://www.ncbi.nlm.nih.gov. Another example of a mathematical algorithm utilised for the comparison of sequences is the algorithm of Myers and Miller, CABIOS (1989). The ALIGN program (version 2.0) which is part of the CGC sequence alignment software package has incorporated such an algorithm. Other algorithms for sequence analysis known in the art include ADVANCE and ADAM as described in Torellis and Robotti (1994) Comput. Appl. Biosci., 10 :3-5; and FASTA described in Pearson and Lipman (1988) Proc. Natl. Acad. Sci. 85:2444-8. Within FASTA, ktup is a control option that sets the sensitivity and speed of the search.

In addition, the present invention provides an expression product of CGB2 or CGB1 for use in the investigation of a cancerous condition.

The present invention provides a kit of parts for detecting and/or measuring the expression product of *CGB2* or CGB1, preferably the 230bp splice variant, comprising an agent for detecting and/or measuring the expression of the 230bp splice variant. The agent for detecting and/or measuring the expression of the 230bp splice variant can include any one or more of a set of primers capable of hybridising to the 230bp splice variant transcript, a probe capable of hybridising to the 230bp splice variant transcript, and an antibody capable of specifically binding to the pre-protein or protein translated from the 230bp splice variant transcript. For example the kit may comprise a forward primer which hybridises to a sequence within Exon 1 of CGB2 or CGB1 or at the Exon1/Exon2 boundary. Preferably the forward primer is 5'-CGTCCAACACCCCTCACTCC-3' (SEQ ID No: 4.) The kit may also comprise a reverse primer which can hybridise to any sequence after the initiation site in Exon 2. Preferably the reverse primer is 5'- 5 GGCAGCCCTCCTTCTCCAC-3' (SEQ ID. NO:5). The kit may further comprise primers or hybridisation probes capable of hybridising with a housekeeping gene, used as a control. Suitable housekeeping genes are known to the skilled person and includes glyceraldehyde-3-phosphate dehydrogenase (GAPDH).

Preferably the kit may further comprise an agent for detecting and/or measuring the expression of the other CG genes CGB3 *CGB5,* CGB7, and/or CGB8. Thus the kit may further comprise primers or hybridisation probes capable of hybridising to the mRNA generated from CGB3 *CGB5,* CGB7, and/or CGB8. Preferably the primers are Forward (5'-CAGCACCTTTCTCGGGTCAC-3') (SEQ ID No:6.) and Reverse (5'-CAGGGAGTAGGGTGTAGGAAGG-3') (SEQ ID No: 7.) The agent for detecting and/or measuring the expression of the other CG genes, CGB3 CGB5, CGB7, and/or CGB8 may also be an antibody capable of specifically binding to the pre-protein or protein produced from the the other CG genes, CGB3 *CGB5,* CGB7, and/or CGB8.

The kit of the invention may further comprise one or more excipients and/or reagents, and materials for carrying out the method of the invention. Such excipients include buffers, reaction solutions, labels, lysing reagents etc.

The kit may further comprise a set of instructions for utilising the kit.

It is thought that the secretion of hCGβ may contribute to the metatasis of the cancerous conditions, and so reducing the level of secreted hCGβ may prevent or inhibit metatasis. Thus in the further aspect the disclosure provides a method of treating an hCGβ secreting cancerous condition comprising administering to patient in need thereof, an pharmaceutically acceptable amount of an agent capable of reducing the level of secreted hCGβ.

In another aspect the invention provides an agent capable of reducing the level of secreted hCGβ for use medicine, in particular in the treatment of an hCGβ secreting cancerous condition. In a related aspect the invention provides a pharmaceutical composition comprising an agent capable of reducing the level of secreted hCGβ and a pharmaceutically acceptable carrier, diluent or excipient.

An agent capable of reducing the level of secreted hCGβ can be an agent that reduces the expression of one or more CG genes, in particular *CG2* and/or *CG1*. Suitable agents include siRNA molecules which specifically bind to sequence of the CG genes, in particular the *CG2* and/or *CG1* gene. Preferably the siRNA are substantially complementary to, or capable of hybridsing to, the 230bp splice variant trasncript. The siRNA molecules may comprise any one or more of the sequences listed in Tables 3 or 4 (SEQ ID Nos 16-31). siRNA, also known as short interfering RNA or silencing RNA, is a class of double stranded RNA molecules that can be used to interfere with the expression of specific gene. siRNA molecules occur in nature, but can also be used experimentally to suppress the expression of a gene *in vitro* or *in vivo.* This may be achieved by targeting an mRNA for degradation, preventing mRNA translation or by establishing regions of silenced chromatin. siRNA molecules are usually short with an average length of 20 to 25 residues. The siRNA molecules themselves can be between 15 and 30 nucleotides in length, optionally 15 and 25 nucleotides in length, such as 15, 16, 17, 18, 19, 20, 21, 22, 23, 24, 25, 26, 27, 28 or 29 nucleotides in length, typically 19 to 29 nucleotides. The target nucleotides and/or the nucleotides of an siRNA are generally contiguous, although siRNA molecules may undergo processing or chemical modification prior to delivery to increase their stability and/or efficacy. Double stranded RNA molecules may also be used that are later processed into smaller siRNA molecules *in vivo,* for example by action of the enzyme Dicer.

"Substantially complementary" refers to at least 50% homology to a sequence that is complementary to the target nucleotide (or segment thereof), for example at least 60%, 70%, 80%, 90%, 95%, 98%, 99% or 100% homologous. For example, a siRNA targetted to the CGB2 and/or CGB1 gene product, such as the 230bp splice variant transcript may be at least 50%, 60%, 70%, 80%, 90%, 95%, 98%, 99% or 100% homologous to a 19 to 29 residue nucleotide sequence that is complementary to a segment of the CGB2 and/or CGB1 gene product such as the 230bp splice variant mRNA. Therefore, CGB2 and/or CGB1 gene product inhibitors such as the230bp splice variant inhibitors that are nucleic acids may have a sequence that is the reverse complement (antisense) to the target nucleic acid or segment thereof.

siRNAs can easily be designed to target specific DNA (or mRNA) sequences according to a number of algorithms known to the skilled person. Such methods are described in, for example, Walton et al., FEBS, 2010, 277(23):4806-13 and Pei et al., Nat Methods, 2006, 3(9)670-6. The siRNA molecules may be chemically modified, for example to increase potency, half-life or stability (for example by optionally adding one or more thymine residues to the end of the siRNA duplex). Such chemical modification is described in, for example, Bramsen & Kjems, Methods Mol Biol, 2011, 721:77-103.

In order to increase the stability of the siRNA, they may be chemically modified. For example, they may be incorporated into vectors, liposomes, vesicles, beads (for example resin or agar beads) or microparticles, or any other encapsulation technology, to assist in their delivery. Incorporation of medically active substances into microparticles is described in WO2006/97725. The vector, liposome, vesicle, bead or microparticle may be targeted to the appropriate tissue by means know to the skilled person.

Other delivery methods will be apparent to the skilled person. For example, lentivirus-expressed siRNA vectors against Alzheimer disease are described in Peng & Masliah, Methods in Molecular Biology, 2010, 614:215-224. RNA interference as a tool for Alzheimer's disease therapy is described in Orlacchio et al., Mini-Reviews in Medicinal Chemistry, 2007, 7(11):1166-76. RNAi-based therapeutic strategies for metabolic disease are described in Czech et al., Nature Reviews Endocrinology, 2011, 7:473-484. Local administration of siRNAs to the eye is described in Reich et al., Molecular Vision, 2003, 9:210-216. Therapeutic silencing of an endogenous gene by systemic administration of modified siRNAs is described in Soutschek et al., Nature, 2004, 432:173-178. siRNA delivery for treatment of muscular dystrophy is described in Hagstrom et al., Molecular Therapy, 2004, 10:386-398.

As described in Dykxhoorn et al, Gene Therapy, 2006, 13:541-552, there are two general ways for delivering siRNA molecules to patients: delivery using double stranded siRNA molecules or delivery using gene therapy to express precursor RNAs from viral vectors. Accordingly, embodiments of the present invention include such strategies, for example double stranded 230bp splice variant mRNA targeted siRNA molecules for use in methods and compositions of the invention. Further discussion on formulation and deliver of siRNA molecules can be found in, for example, Burnett et al., Biotechnol J., 2011, 6(9):1130-46, Peer et al., Gene Ther., 2011, 18(2):1127-33, Manjunath & Dykxhoorn, Discov. Med., 2010, 9(48):418-30 and Zheng et al., Proc Natl Acad Sci USA, 2012, 109(30)11975-80.

Once delivered, siRNA molecules may target mRNA (such as mRNA encoding the 230bp splice variant mRNA) for degradation or may prevent their translation, thereby silencing gene expression. Precursor RNA may be processed by action of the enzyme Dicer, which cleaves long double-stranded RNA (dsRNA) molecules into short double stranded fragments suitable for use in siRNAs and miRNAs. Accordingly, when carrying out the present invention, a skilled person may use a suitable precursor that is processed *in situ* (*in vivo*) by action of the enzyme Dicer into a suitable and effective siRNA molecule. In some embodiments, siRNAs may target genomic DNA and prevent or interfere with transcription of a gene.

The half-life of siRNA molecules can be extended by, for example, incorporating the siRNA into particles (to avoid renal filtration) and by chemically modifying the sugar backbone. For example the siRNA may be 2'-O-methyl or 2'-fluoro substituted, or it may be modified at the 2' position to 2'deoxyribose. siRNA molecules may be conjugated to lipids, such as cholesterol, to increase their half-life, or they may be incorporated into microparticles or liposomes, and other techniques will be apparent to the skilled person.

The siRNA of the invention may be administered to a patient with separate, simultaneous or subsequent delivery of suitable transfection reagents, for example during local delivery to tissues such as the lung, vagina, a subcutaneous tumour, muscle, eye or the nervous system. Systemic delivery may be achieved by, for example, rapid high-pressure intravenous injection ("hydrodynamic therapy") or receptor-mediated systemic delivery. An in-depth review is provided in Dykxhoorn *et al.*

Alternatively the agent capable of reducing the level of secreted hCGβ can be a compound capable of binding specifically to the secreted hCGβ. Preferably the agent is an antibody or lectin capable of binding to the secreted hCGβ, such as CMAhCGβ. Suitable antibodies include those as described herein.

The compositions of the invention may be presented in unit dose forms containing a predetermined amount of each active ingredient per dose. Such a unit may be adapted to provide 5-100mg/day of the compound, preferably either 5-15mg/day, 10-30mg/day, 25-50mg/day 40-80mg/day or 60-100mg/day. For compounds of formula I, doses in the range 100-1000mg/day are provided, preferably either 100-400mg/day, 300-600mg/day or 500-1000mg/day. Such doses can be provided in a single dose or as a number of discrete doses. The ultimate dose will of course depend on the condition being treated, the route of administration and the age, weight and condition of the patient and will be at the doctor's discretion.

The compositions of the invention may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier(s) or excipient(s).

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical formulations adapted for topical administration may be formulated as ointments, creams, suspensions, lotions, powders, solutions, pastes, gels, sprays, aerosols or oils.

For applications to the eye or other external tissues, for example the mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical formulations adapted for topical administration to the eye include eye drops wherein the active ingredient is dissolved or suspended in a suitable carrier, especially an aqueous solvent.

Pharmaceutical formulations adapted for topical administration in the mouth include lozenges, pastilles and mouth washes.

Pharmaceutical formulations adapted for rectal administration may be presented as suppositories or enemas.

Pharmaceutical formulations adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical formulations adapted for administration by inhalation include fine particle dusts or mists which may be generated by means of various types of metered dose pressurised aerosols, nebulizers or insufflators.

Pharmaceutical formulations adapted for vaginal administration may be presented as pessaries, tampons, creams, gels, pastes, foams or spray formulations.

Pharmaceutical formulations adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

Preferred unit dosage formulations are those containing a daily dose or sub-dose, as herein above recited, or an appropriate fraction thereof, of an active ingredient.

It should be understood that in addition to the ingredients particularly mentioned above, the formulations may also include other agents conventional in the art having regard to the type of formulation in question, for example those suitable for oral administration may include flavouring agents.

An embodiment of the invention will now be described in the following examples which refer to the following figures:
Figure 1 provides a Diagrammatic representation of the hCGβ gene cluster and comparison of different hCGβ genes.

**A.** *hCGβ-LHβ* gene cluster; diagrammatic representation showing relative positions of the CGB genes, *LHB* and *SNARs.* Straight arrows show direction of transcription, and open arrowheads represent SNAR-G genes. Start codons - *ATG₁ and ATG₂* correspond to alternative open reading frame.

**B***:* Schematic of *CGB1* and *-2* genes compared to *CGB*, *CGB5*, *CGB7*, *CGB8* gene structures, illustrating exon and the intron regions of the *CGB* genes and indicating the potential three main splice variants reading through into intron 1 (+47 bp, +166 bp, +167 bp). Translated regions are depicted as black boxes; open reading frames are shown as open boxes. *SNAR-Gs* are located in the upstream region of exon 1 of genes CGB1 and -2 and are shown as a smaller black box along the gene line.

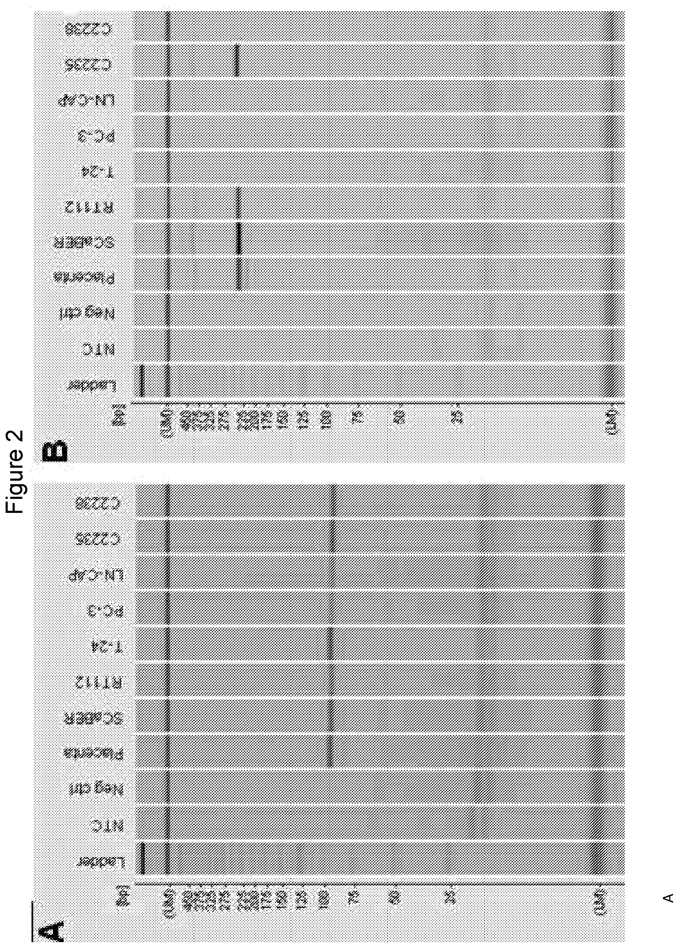

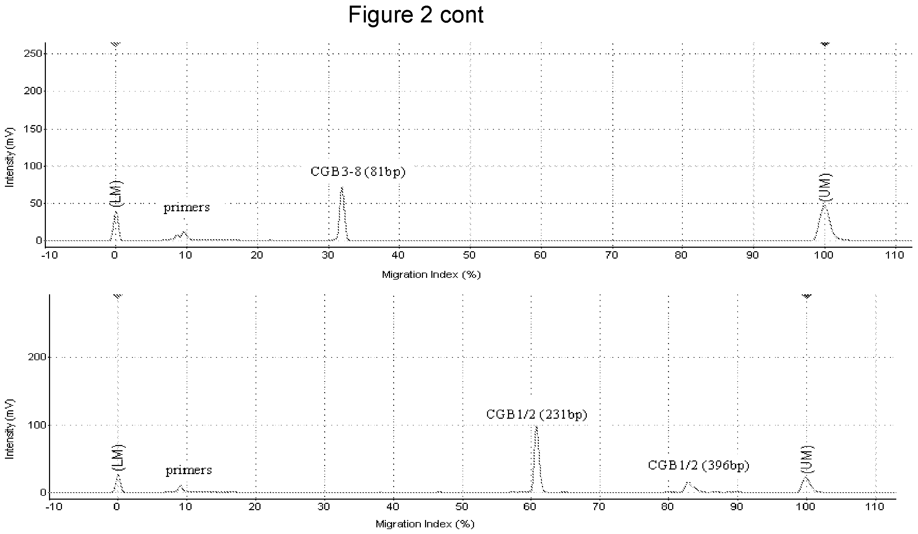

Figure 2 shows Electrophoresis and electropherogram of qPCR products on Microchip Electrophoresis System MultiNA. Separation of real-time PCR products and analysis by electrophoresis system MultiNA. **A:** Amplification of classical CGB genes by quantitative PCR. The desired product is seen in appropriate lanes: placenta, SCaBER, RT112, T24, C2235 and C2238. **B:** PCR products obtained during amplification of genes *CGB1* and *-2*. The product 231 bp corresponds to calculated specific product of CGB1 and -2, whilst the primers are the bands below 25 bp. In addition +166 bp splicing variant was amplified (with calculated by MultiNa software mass 396 bp). The position of upper (UM) and lower (LM) marker dyes of the MultiNA separation are indicated, as is the position of the base pair reference mass markers. The ladder (Invitrogen) shows the bands between 25 bp and 450 bp. Any slight difference in size of product is caused by variations in separation on different microchips. Non template control (NTC) and negative control (Neg ctrl) are indicated. Representative electropherograms from MultiNA electrochip system of separated PCR products amplified **(A)** with *CGB, CGB5,-7* and -8 primers with a mass of 81 bp **(B)** and with CGB1 and -2 specific primers with predominant product with a mass of 231 bp (calculated 229 bp), and less abundant 396 bp. LM and UM were used for calibration according to the manufacturer. The axes of the plot indicates migration index (x) and fluorescence intensity (y). Each peak in the electropherogram represents a DNA fragment of PCR product or primer which migrates according to molecular weight. Migration index is inversely proportional to the length of DNA fragments and is used by software to calculate exact mass.

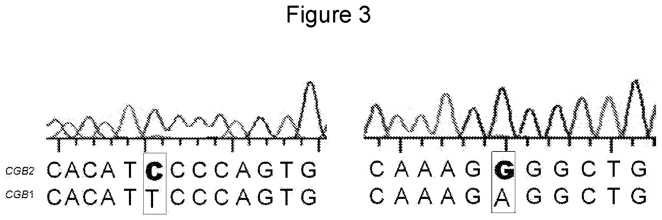

Figure 3 shows Sequencing analysis of *CGB1* and *-2* qPCR products. Sections of specific sequencing of *CGB1* and *-2* PCR products (both 231 bp and 396 bp) were sequenced indicating the single marker nucleotide differences between *CGB1* and *CGB2* mRNA (at nucleotides 185 and 223 - accession numbers NM_033377.1 and NM_033378.1 respectively). This demonstrates that expression of *CGB2 mRNA* is the predominant source of *CGB1*/*2* RT-PCR product detected in qPCR with *CGB1*/*2*-specific primers.

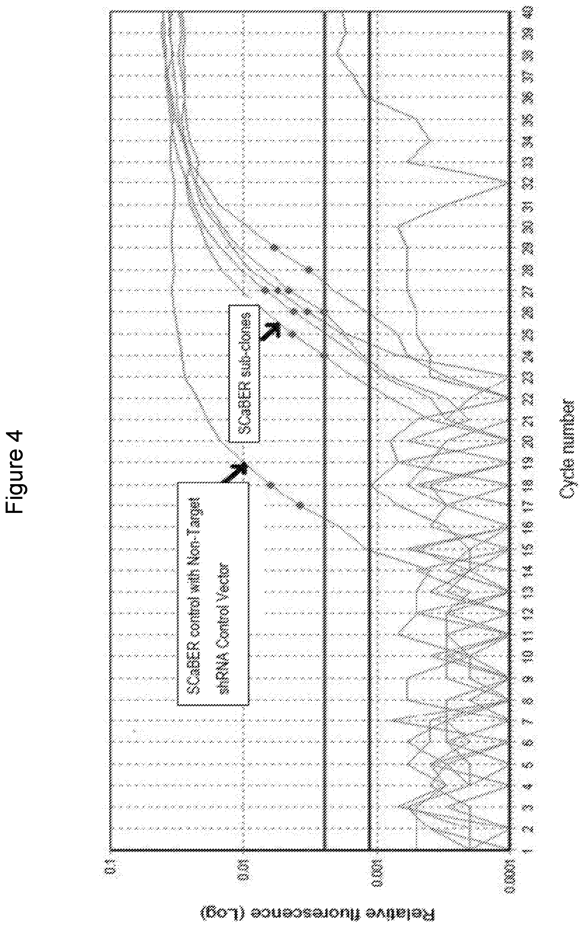

Figure 4 shows the Real-time PCR amplification curves to establish crossing points (Cp). Amplification of control clone SCaBER (CGB) gene expression following transfection with Non-Target shRNA Control Vector in comparison to the SCaBER clones stably transfected with shRNA targeting CGB gene. The control clone corresponds to Cp of 16.44, The lowest Cp (23.95) of the CGB-targeted clones corresponds to SCaBER clone 1 and the highest Cp (27.71) corresponds to SCaBER clone 2.

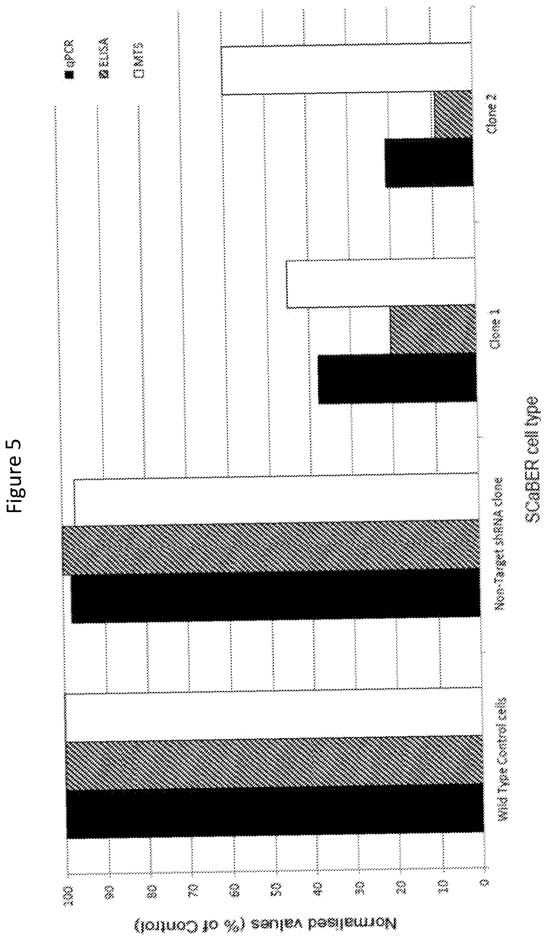

Figure 5 shows the Effect of human chorionic gonadotropin (CGB) knockdown by gene-specific short hairpin RNA (shRNA) lentiviral particles. Relative level of (CGB) mRNA measured by real-time PCR (qPCR) after silencing with (shRNA) target construct, amount of hCGβ in the culture medium estimated by ELISA and number of viable cells measured by MTS. Data were normalised to the level set at 100% measured for non-target shRNA transduced cells. A decreased mRNA level of (CGB) transcript to 38% in clone 1 and 21% in clone 2 was detected by real-time PCR. Level of hCGβ was reduced for clone 1 (to 20.45% of control) and clone 2 (to 9.09% of control). Cancer cell number was reduced in clone 1 by 55% and in clone 2 by 40%.

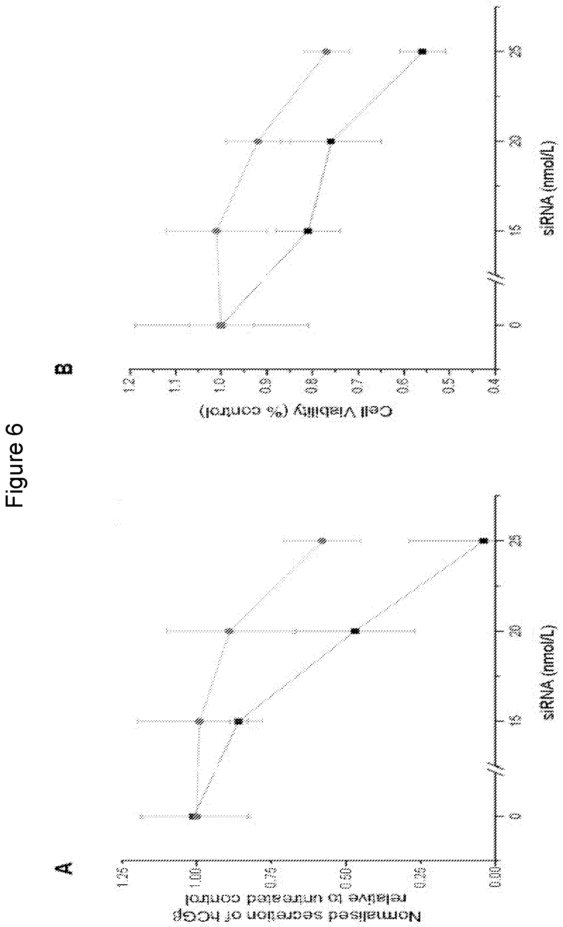

Figure 6 shows the Effect of silencing of CGB1 and CGB2 and classical CGB genes on bladder cancer cell line SCaBER.

6A: Secretion of hCGβ into culture media of SCaBER cancer cells treated with increasing concentrations of siRNA (targeting *CGB1* and *CGB2*, or *CGB*, *CGB5*, *-7* and *-8*). Values are expressed as percentage of untreated control and each point is the mean of six replicates of the assay run in duplicate; the bars represent standard deviation about the mean. EGFP silencing represents non-specific effects of introducing a non-functioning siRNA.

6B: Influence of RNA silencing on SCaBER cell viability - increasing concentration of siRNA (nmol/l) and relative level of viable cells are shown. MTS assay values are expressed as a percentage that of untreated control. Results are the mean from six replicates of the assay. The bars represent standard deviation about the means. EGFP silencing represents non-specific effects of introducing a non- functioning siRNA.

It is to be understood that the various features that are described in the following and/or illustrated in the drawings are preferred but not essential. Combinations of features described and/or illustrated are not considered to be the only possible combinations. Unless stated to the contrary, individual features may be omitted, varied or combined in different combinations, where practical.

### Example 1: CGB2 and CGB1 gene expression

The human bladder cancer cell lines RT112, SCaBER and T24; breast cancer cell lines C2235 and C2238; prostate cancer LN-CAP and PC-3, were used. Third trimester placenta was used as a positive control.

The cell lines were obtained from American Type Culture Collection (Manassas, USA) and from the European Collection of Animal Cell Cultures (Porton Down, Dorset, UK). Cells were cultured in RPMI-1640 medium (Invitrogen) supplemented with 10% fetal bovine serum (FBS) (Invitrogen) with 5% antibiotics at 37°C in 5% CO₂.

Total RNA was isolated from all cell lines as well as normal term placental tissue (placenta obtained after vaginal delivery in 38th week of pregnancy - positive control), and fresh water shrimp (negative control) using SV Total RNA Isolation System (Promega,UK). 1µg of total mRNA was used for first-strand synthesis cDNA by Verso cDNA Kit using oligo-dT primer and random hexamers in a 1:3 ratio (Thermo Scientific, UK).

Relative quantification of CGB gene expression for the selected breast, bladder and prostate cancer cells lines and placental tissues was carried out in Quantica thermocycler (Techne, Barloworld Scientific Ltd) using ABsoluteTM Blue QPCR SYBR^{®} Green Mix kit (Thermo Scientific) with ROX passive reference dye as recommended by the manufacturer. PCR amplification was carried out in a total volume of 25 µl, containing 1× SYBR^{®} Green mix (3 mM MgCl₂, 100 nm ROX, 0.2 mM dNTPs, 1 U Taq DNA polymerase), 70 nM of specific forward and reverse primers (Table 1) and 2µl of cDNA sample. The amplification program consisted of 1 cycle of 95°C 10 min, followed by 40 cycles of 95°C for 10sec, annealing 60°C for 5sec, and 72°C for 9sec. After termination of 45 cycles, a dissociation curve analysis was performed to confirm melting temperatures of expected products.

All primers were designed in NCBI/Primer3 - BLAST tool and purchased from Sigma (Sigma Life Science). One set of primers were specific for *CGB1*/*2*:
forward primer (5'-CGTCCAACACCCCTCACTCC-3') (SEQ. ID NO: 4) designed to anneal to nucleotides 118-137 in mRNAs of human chorionic gonadotropin, beta polypeptide 1 (*CGB1*) and mRNA of human chorionic gonadotropin, beta polypeptide 2 (CGB2) (GeneBank accession no. NM_033377 and NM_033378, respectively), and reverse primer (5'-GGCAGCCCTCCTTCTCCAC-3') (SEQ. ID NO: 5) to nucleotides 329-347 of mRNAs. This set of primers span intron 1 and all major splicing variants, which have been described before in placenta, should be detected.

The second set of primers (F 5'-CAGCACCTTTCTCGGGTCAC-3' (SEQ. ID NO: 6) and R 5'- CAGGGAGTAGGGTGTAGGAAGG-3') (SEQ. ID NO: 7) were specific for CGB, CGB5, CGB7 and CGB8 mRNAs designed to anneal to nucleotides 10-30 and nucleotides 73-95 in mRNA (GeneBank accession no. NM_033183).

In order to check the integrity of the reverse transcriptase reaction the expression of the housekeeping gene encoding glyceraldehyde-3-phosphate dehydrogenase (GAPDH) amplified using a specific primer set (F 5'-CATGGGTGTGAACCATGAGAAG- 3' (SEQ. ID NO: 8) and R 5'-GTGCTAAGCAGTTGGTGGTGC-3' (SEQ. ID NO: 9)) was used as an endogenous control (GeneBank accession no. NM_002046). NTC (Non Template Control) contained complete qPCR master mix but no cDNA. As a negative control we used non-human cDNA from shrimp. All experiments were performed in triplicates and mean 20 crossing point (Cp) values were calculated.

Real-time PCR products we detected by Microchip Electrophoresis System MCE202 MultiNA (Shimadzu) according to manufacturer instructions for DNA-500 kit (Shimadzu). The length of DNA products was estimated based on separation of 25bp DNA ladder (Invitrogen). Applying MultiNA electrochip system enabled very precise size estimation of qPCR products by MultiNA Viewer software. In addition, to fully identify the qPCR products, they were separated on 1% agarose gel, cut out and purified with PureLinkTM Quick Gel Extraction Kit (Invitrogen), followed by sequencing (forward and reverse direction) by GATC Biotech (London, UK). Blast sequence analysis was performed on generated sequences to identify homologies with those deposited in NCBI gene databases.

For the calculation of relative quantity of CGB genes we applied ΔΔCp method described by Livak and Schmittgen . The level of transcription of CGB1/2 and CGB, CGB5, CGB7, *CGB8* in studied cancer cell lines was normalised on the basis of the level of its housekeeping gene GAPDH content and relatively calculated to the level of the gene expression in placental tissue (calibrator). Mean crossing point value was used in calculations. Final results are expressed as N-fold differences in CGB expression relative to calibrator gene expression level equal to 1.0.

The media from confluent cell cultures grown on 75cm² flasks was collected and assayed to estimate the amount of secreted protein by free hCGβ assay. Cell numbers were estimated using a haemocytometer following trypsin-EDTA liberation from the flask.

A two-site FBT-11 immunoenzymometric assay, described previously, for the highly specific detection of free beta subunit of hCG was used. This system utilizes a unique mouse monoclonal anti-hCGβ antibody - FBT11 - directed against epitope β6/7 of hCGβ as capture antibody and rabbit anti-hCGβ conjugated with horseradish peroxidise (4001-POD), which is a core antibody recognising the β1 epitope, for detection. A solution of TMB Reagent (Sigma) diluted in deionised water (1:1), was used for quantification of peroxidase and produced a blue product. The reaction was stopped with the addition of 2N HCI and absorbance was measured spectrophotometrically at 450nm on a FLUOstar OPTIMA (BMG Labtech). The assay was calibrated each time against a standard curve of recombinant hCGβ (Sigma) at serially diluted concentration range of 50ng/ml to 0.5ng/ml, which had been calibrated against the international reference preparation of hCGβ (NIBSC, Potters Bar, UK). Intact hCG concentrations were quantified using the USA hCG Reference Service "in house" intact hCG ELISA utilising the antibody combination of McAb 2119 with 4001-POD.

### Results

The hCGβ gene cluster was separated into two classes of genes: CGB, CGB 5,7,8 and CGB 1/2 using sequence specific primers we amplified genes independently to quantify expression levels. No product appeared to be derived from genomic DNA as no band corresponding to predicted products of 481 or ∼629bp (containing the entire intronic sequence) respectively, were amplified.

In all bladder cancer (SCaBER, T24, RT112); breast cancer (C2235, C2238) and prostate cancer (LN-CAP, PC-3) cell lines tested, transcripts for CGB,CGB5,7&8 were detected (Fig. 2-A). The placental control tissue also revealed the presence of CGB, CGB5,7,8 transcripts. The normalised values of average crossing point (Cp) differ between cancer cells and indicates different expression level of CGB genes in selected cancer cell lines. Separation of the qPCR products on the MultiNA system confirmed the presences of the predicted PCR products with calculated mass (Fig. 2 - B). The transcripts of CGB1,2 genes were found only in bladder cancer cell lines SCaBER and RT112 and breast cancer cell line C2235 (Fig. 2- A). All qPCR products were separated by MultiNA electrophoresis, where a 231bp product was detected (a 229bp product was predicted). In addition in these cell lines a less abundant high molecular size product of 396bp was detected for CGB1/2 qPCR (Fig. 2-B). The products (81bp, 229bp, 396bp) were sequenced and align with known CGB GenBank sequences database.

The 81bp product arising from *CGB(3),5,7,8* qPCR was aligned with the known mRNA and predicted sequences for these CGB genes. For the 229bp and 396bp qPCR product for CGB1/2 amplification sequence analysis identified that the product was arising from expression of CGB2 (see figure 3) as these vary by two nucleotides at positions 185 and 223 (accession numbers NM_033377.1 & NM_033378.1 respectively for genes 1 and 2). The predominant nucleotides detect at these sequence positions were C and G of CGB2 (GeneBank accession no. NM_033378.1).

The less abundant high molecular sized qPCR product (396bp - Figure 2- B), correspond to one of the splicing variant of genes CGB1/2 described previously in testes and placenta. The presence of this splicing variant includes an additional 166 base pairs from intron 1 (165 as determined by the MultiNA separation system). This larger fragment revealed base pair coding corresponding to the predicted inclusion region of intron-1 nucleotide sequences of the CGB genes.

The level of CGB genes was quantified and compared to the expression level of CGB genes in term placenta. *CGB, CGB5,7,8* genes among our tested samples were the highest in the placental tissue. Expression of *CGB*, *CGB5,7,8* mRNA by cancer cell lines varied but was in most cases was less than 1% of that seen by placental expression. Expression by the bladder cancer cell line T24 was 1.6%, SCaBER 3.1% and by the two breast cancer cell lines 6% (C2335) and 25% (C2238), of that seen in the placental control tissue (See table). mRNA from genes CGB1/2 was detected in two out of three bladder cancer cell lines; SCaBER and RT112 showed significantly higher level of transcripts in comparison to term placenta, both 128 fold higher. The breast cancer cell line C2335 demonstrated a 32 fold higher expression of CGB1/2 mRNA compared to placenta control sample (See table 1). The presence and amount of free hCGβ protein secreted into the culture medium was measured by specific hCGβ ELISA. The highest level of hCGβ protein in medium was seen in the bladder cancer cell line SCaBER (4.4ng/106 cells/24h), breast cancer cell line C2235 (2.3ng/106 cells/24h) and bladder cancer cell line RT112 (0.78ng/106 cells/24h). In the remaining cancer cell lines (T24, PC-3, LN-CAP, C2238) no hCGβ could be detected in the medium (< 0.5ng/106 cells/24h) using this method. No intact hCG was detected in any culture medium (see Table 1).

### Example 2 - siRNA directed silencing of hCGβ expression

### Method 1 - all hCGβ approach, stable protein knockdown

Lentiviral-based shRNAs that generate small interfering RNAs (siRNAs) were used to transduce hCGβ secreting cancer cells. Two different shRNA gene specific constructs targeting different exonic regions of CGB genes were used in these studies. Stable gene silencing was established by puromycin resistance clone selection. Reduction in the level of CGB mRNA was measured by real-time polymerase chain reaction (PCR) and protein detection by enzyme-linked immunosorbent assay (ELISA). The effect on cell population growth was then estimated by MTS (tetrazolium salt reduction) assay.

shRNA lentiviral transduction. MISSION^{®} shRNA Lentiviral Transduction Particles (Sigma Aldrich, Pool, UK) were used to knockdown the CGB in hCGβ-expressing bladder cancer cell line SCaBER, derived from squamous cell carcinoma of the human urinary bladder (ATCC, Rockville, Maryland, USA) which have been studied previously. TRC1-pLKO.1-puro vector containing a hairpin insert with gene specific sequence was used for cancer cell transduction according to the manufacturers' protocol in addition to hexadimethrine bromide (8 µg/ml) to enhance transduction efficiency. Sequences of inserts in shRNA constructs targeting CGB gene (exon 2 or 3) (Acc. No. NM033043) are shown in Table 3. Stable gene knockdown was established by cellular resistance to puromycin (500 ng/ml). Clones were isolated and several sub-clone cell lines were established. The Non-Target shRNA Control Vector (Sigma Aldrich, Pool, UK) containing an insert sequence that does not target any human gene but can activate RNA-induced silencing complex (RISC) and the RNAi pathway served as a negative control. In addition, MISSION^{®} Control Vector pLKO.1-puro (no shRNA insert) (Sigma Aldrich, Pool, UK) along with untreated wild-type cells were used. RNA extraction and cDNA synthesis. Quantitative real-time PCR Total RNA was extracted from the selected cell clones using SV Total RNA Isolation System (Promega, Southhampton, UK). One microgramme of total mRNA was used for first-strand synthesis cDNA by Verso cDNA Kit (Thermo Scientific, Leicestershire, UK). CGB transcript quantity was determined by real-time PCR (Quantica, Techne, Stone, UK); using specific primers designed for amplification of any CGB genes coding hCGβ protein (NM_033043): forward primer 5'-TCACCGTCAACACCACCATCT-3' SEQ. ID NO: 32 and reverse primer 5'-ATGGAGTCGAAGCGCACATC-3' SEQ. ID NO: 33 and the level of the housekeeping gene, glyceraldehyde 3-phosphate dehydrogenase (GAPDH; NM_002046) forward primer 5'-CATGGGTGTGAACCATGAGAAG-3' (SEQ. ID NO:8) and reverse primer 5'-GTGCTAAGCAGTTGGTGGTGC-3' (SEQ. ID NO:9). Real-time PCR was carried out according to the protocol for ABsoluteTM Blue QPCR SYBR^{®} Green Mix kit (Thermo Scientific). NTC (Non Template Control) contained complete qPCR master mix but no cDNA template. As a negative control we used non human cDNA from shrimp. All experiments were performed in triplicates and mean crossing point (Cp) values were calculated.

Relative quantification of the level of CGB mRNA transcripts. Relative quantification of CGB gene expression was calculated using the ΔΔCp method described by Livak and Schmittgen. The level of transcription of CGB genes was normalised against the level for the housekeeping gene GAPDH and calculated relatively to the level of the gene expression in the cells transduced with Non-Target shRNA Control Vector (calibrator) using average crossing point values. Final results are expressed as percentage differences in CGB expression relative to calibrator gene expression set at 100%.

hCGp-specific ELISA. Synthesis of hCGβ and release into culture media was determined by specific free hCGβ ELISA utilising monoclonal antibody FBT11 directed against epitope β6/7 of hCGβ and capture antibody - rabbit anti-hCGβ conjugated with horseradish peroxidise (4001-POD), which is a core antibody recognising β1 epitope. The assay has been validated and described previously. Standards used here were recombinant hCGβ (Sigma) these were calibrated against 1st International Reference Preparation (IRP) for hCGβ - (NIBSC, Potters Bar, UK) in a concentration range from 0.5 ng/ml to 50 ng/ml. The media from confluent cell cultures grown on 75 cm² flasks were collected and assayed to estimate the amount of secreted protein normalised to 1×10⁶ cells.

Cell proliferation (MTS) assay. For viability assays 100 µl of complete growth medium in the cell culture wells was replaced with 20 µl of CellTiter 96^{®} AQueous^{®} One Solution Cell Proliferation Assay reagent (Promega). The plate was incubated at 37°C in humidified atmosphere with 95% air, 5% CO₂ for 1―4 hours until colour was well developed and the absorbance was then measured at 490 nm on a Fluostar OPTIMA (BMG Labtech, Aylesbury, UK). Data was normalised against the optical density achieved for the control (set at 100%) and expressed as a percentage change in cell number.

### Results

Effect of stable CGB gene silencing on protein expression and cell growth. Real-time PCR analysis of clones showed about 60%-80% (clone 1 and clone 2 respectively) reduction in the level of hCGβ mRNA transcripts when compared to SCaBER cells transduced with the Non-Target shRNA Control Vector (Figure 4). Similarly, the hCGβ protein concentration in culture media for clone 1 and clone 2 was reduced to 20% and 9% respectively. hCGβ concentration in exhausted culture media was reduced to 0.4 and 0.9 ng/ml compared to 4.4 ng/ml in medium from wild type SCaBER cells over the same period and under the same conditions. After 72 hours in culture, populations of sub clone cells, with stable knockdown of CGB expression, were reduced when compared with wild-type SCaBER cells as measured by MTS assay (Figure 5). There was less than 5% difference in the level of hCGβ transcripts, protein secretion and growth of cells between control (non-infected cells) and cells exposed to control vector non-target shRNA (see Figure 5).

### Method 2 - Specific CGB1/2 approach, transient protein knockdown

The siRNA duplexes (Sigma-Aldrich, Gillingham, Dorset, UK) were designed primarily to target mRNA arising from the published sequences for CGB2 (GenBank accession no. NM_033378) and CGB8 (GenBank accession no. NM_033183). These duplex sets were distinct in target but CGB2 siRNA duplexes cross-hybridized significantly (100%) with CGB1, and the CGB8 siRNA duplexes significantly cross-hybridized (>95%) with all classical CGB genes (Table 5). Bladder cancer cell line SCaBER cells were transfected with siRNA duplexes using CodeBreaker^{™}siRNA Transfection Reagent (Promega, UK) according to the manufacturer's protocol. A total of 5×10³ cells per well were plated into 100 µl complete growth medium in 96-well plates (BD Falcon, Oxford, Oxfordshire, UK) and allowed to grow for 24 hours (until they were 50% confluent). Cells were then transfected with siRNA at a final concentration of 15 nM, 20 nM and 25 nM of siRNA in sets of six replicates. Plates were incubated for a further 72 hours at 37°C and 5% CO₂. A non-specific siRNA targeting enhanced green fluorescent protein (EGFP) was included as a transfection response control. Negative controls were cells which had been treated with the transfection reagents (CodeBreaker^{™}siRNA ; Promega), but without any siRNA.

The media from confluent cell cultures grown in 75 cm² flasks from before and after specific CGB gene silencing was harvested for hCGβ protein quantification. hCGβ concentration was estimated using a two-site FBT-11 immunoenzymetric assay, described previously. Cell numbers, post culture, were estimated using a haemocytometer and used to normalise protein concentrations (1×10⁶ cells). The assay was calibrated against a standard curve of recombinant hCGβ (Sigma) (range of 50 pg/l to 0.5 pg/l) which had been calibrated against the first international reference preparation of hCGβ (batch 75/551; NIBSC, Potters Bar, UK). Intact hCG concentrations were quantified using the USA hCG Reference Service in-house intact hCG ELISA, utilising the antibody combination of McAb2119 with 4001-POD. This was calibrated against the 3rd International Standard preparation of hCG (batch 75/589; NIBSC). The limit of detection (lowest standard) for the intact hCG assay was 0.75 pg/I.

For viability assays, cells were seeded in complete growth medium as described above. Growth medium was replaced with 20 µl of CellTiter 96^{®} AQueous^{®} One Solution Cell Proliferation Assay reagent (Promega) and cells were incubated at 37°C for 1-4 hours until colour was well developed in a humidified atmosphere with 95% air, 5% CO2. 3-[4,5,dimethylthiazol-2-yl]-5-[3-carboxymethoxy-phenyl]-2-[4-sulfophenyl]-2H-tetrazolium (MTS) is a tetrazolium salt reduction-type assay method - absorbance at 490 nm was measured on a Fluostar OPTIMA (BMG Labtech, Aylesbury, Buckinghamshire, UK), and optical density was measured and expressed as percentage change relative to control cultures in the 96-well plates. Data were normalised against the optical density achieved for the untreated controls, and expressed as a percentage change in cell number. Data were then pooled between plates and expressed as mean and standard deviation change in normalised cell populations from quintuplicate experiments. Statistical significance was conducted using Stats DirectTM (Altrincham, Cheshire, UK) software in ANOVA, or Friedman's two-way analysis (data were not normally distributed).

### Results - Example 2 Method 2

Following siRNA control transfection, secreted hCGβ protein levels ranged from 3-4.6 pg/l for bladder cancer cells. siRNA CGB2 oligo duplex (25 nM) reduced secretion by 96%, while CGB8 oligo duplex reduced secretion by 42% of controls. At all concentrations, we observed that siRNA duplexes effectively inhibited CGB expression (Figure 6A). We further examined whether viability of cells in culture could be changed after silencing to determine the impact of hCGβ secretion on survival. At all concentrations of siRNA, CGB2 oligo-duplex reduced cell numbers significantly: 25 nM by 44% (p<0.0001), 20 nM by 24% (p=0.0011), and 15 nM by 19% (p=0.02) that of the control (Figure 6B). In contrast, the control siRNA directed against the sequence of an unrelated protein (EGFP) failed to produce any significant reduction in relative cell numbers, with reduction of only 13% (p=0.22). When CGB8 gene was targeted by siRNA oligo-duplex, only the highest concentration (25 nM) resulted in a significant reduction of cell growth by 23% that of the control (p=0.0004) (Figure 6B). Non-specific silencing of control EGFP sequence had no effect on cell numbers (1.01%, p=0.42).

Table 4 provides detail of siRNA sequences used for silencing CGB genes. siRNA sequences used for silencing CGB2 (and 1) and CGB8 (and CGB, CGB5, -7 and -8); their location on mRNA mapping to base-pairs on exon 1 (according to GenBank accession number NM_033378 for *CGB2* and NM_033183 for *CGB8*, respectively) and the degree of homology between the siRNA duplexes and the different *CGB* gene sequences.

In this specification, the word "preferable" (or any of its derivatives) indicates one feature or more that is preferred but not essential.

All or any of the features disclosed in this specification (including any accompanying claims, abstract and drawings), and/or all or any of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s).

**Table 1**

| **Cell line or tissue** | ***Normalised Cp CGB(3),5,7,8*** | ***Normalised Cp CGB1*/*CGB2*** | ***Placental Relative Level CGB(3),5,7,8*** | ***Placental Relative Level CGB1,2*** | **Immuno-staining for hCGβ** | **Secretion of hCGβ (ng/1×10⁶ cells/24h)** | **Secretion of hCG (ng/1×10⁶ cells/24h)** |
|---|---|---|---|---|---|---|---|
| **Placenta** | 22 | 34 | 1 | 1 | **+ + +** | - | - |
| **SCaBER** | 27 | 27 | 0.0313 | 128 | **+** | **4.4** | **<0.5** |
| **RT112** | 30 | 27 | 0.0039 | 128 | **+ +** | **0.78** | **<0.5** |
| **T24** | 28 | ND | 0.0156 | ND | **+ + +** | **<0.5** | **<0.5** |
| **PC-3** | 31 | ND | 0.002 | ND | **+ + +** | **<0.5** | **<0.5** |
| **LN-CAP** | 30 | ND | 0.0039 | ND | **+ +** | **<0.5** | **<0.5** |
| **C2235** | 26 | 29 | 0.0625 | 32 | **+** | **2.3** | **<0.5** |
| **C2238** | 24 | ND | 0.25 | ND | **+ + +** | **<0.5** | **<0.5** |

**Table 2 Peptide sequence differences (italics) and homology (bold) between CGB3, CGB5 CGB7, CGB8 mRNA CGB2/1 230 splice variant if translated.**

| Gene variant | Pre peptide | Mature peptide |
|---|---|---|
| CB5,CGB3,CGB7 and CGB8 correctly spliced at exon 1 to exon 2 boundary. Peptide sequence | *MEMFQ***GLLLLLLLSMGGTWA** | **SKEPLRPRCRPINATLAVEKEGC....(remaining mature peptide sequence -** |
| | | PVCITVNTTICAGYCPTMTRVLQGVLPALPQVVCNYRDVRFESIRLPGC PRGVNPVVSYAVALSCQCALCRRSTTDCGGPKDHPLTCDDPRFQDSS SSKAPPPSLPSPSRLPGPSDTPILPQ) |
| CGB2 230bp splice variant identified: peptides if translation initiated at first ATG | *MSTSPVLAEDIPLRERHVKG AAAVAAAEHG RDMGIQGAAS ATVPPHQCHP GCGEGGL*.........(continued mismatch due to frame shift).. | |
| CGB2 230bp splice variant identified: Peptides if translation initiated at 2^{nd} (consensus) ATG | *MSK***GLLLLLLLSMGGTWA** | **SKEPLRPRCRPINATLAVEKEGC...... (Remaining mature peptide sequence match)..** |

### Table 3

A short hairpin RNA (shRNA) which targets all human chorionic gonadotropin (CGB) genes designed against a CGB5 was constructed within a lentivirus vector pLKO.1-Puro. The inserted hairpin sequence contains gene-specific target sequence (highlighted in grey) and loop region which correspond to sequence CTCGAG.

| Table 3 | | |
|---|---|---|
| **shRNA lentiviral particles and clone number** | **Sequence targeting all *CGB* genes inserted into pLKO.1-puro vector** | **Target gene and exon position of sense and antisense sequences** |
| TRCN0000082824 clone 1 | GTGGTGTGCAACTACCGCGATCTCGAGATCGCGG TAGTTGCACACCAC | CGB5 mRNA NM_033043 exon 3 |
| | | Sense strand: nt 588-608 |
| | | Antisense strand: nt 588-608 |
| TRCN0000082826 clone 2 | CCGTGTGCATCACCGTCAACACTCGAGTGTTGAC GGTGATGCACACGG | CGB5 mRNA NM_033043 exon 2 |
| | | Sense strand: nt 496-515 |
| | | Antisense strand: nt 517-503 |

**Table 4**

| **Target gene** | | | **Between-gene homology (%)** | | | | | |
|---|---|---|---|---|---|---|---|---|
| **Nucleotide position in exon 1** | **siRNA duplex Sequence** | | ***CGB* 1** | ***CGB* 2** | ***CGB* 3** | ***CGB* 5** | ***CGB* 7** | ***CGB8*** |
| CGB 2 | Sense CAGUGCUUGCGGAAGAUAU dTdT | 1: | **100** | **100** | 37 | 37 | 37 | 42 |
| position : 188 | Antisense AUAUCUUCCGCAAGCACUG dTdT | 1: | | | | | | |
| CGB 2 | Sense CACGGAGACUCAAUUUACU dTdT | 2: | **100** | **100** | 42 | 37 | 37 | 37 |
| position : 152 | Antisense AGUAAAUUGAGUCUCCGUG dTdT | 2: | | | | | | |
| CGB 2 | Sense CGCUAAGAGAGAGACAUGU dTdT | 3: | **100** | **100** | 42 | 42 | 42 | 42 |
| position : 209 | Antisense ACAUGUCUCUCUCUUAGCG dTdT | 3: | | | | | | |
| CGB 8 | Sense UCACUCCAGCAUCCUACAA dTdT | 1: | 28 | 28 | 84 | **95** | **100** | **100** |
| position : 280 | Antisense UUGUAGGAUGCUGGAGUGA dTdT | 1 : | | | | | | |
| CGB 8 | Sense AGGUUUAAAGCCAGGUACA dTdT | 2: | 42 | 42 | **95** | **95** | **95** | **100** |
| position : 322 | Antisense UGUACCUGGCUUUAAACCU dTdT | 2: | | | | | | |
| CGB 8 | Sense AGUCUCUGAGGUCACUUCA dTdT | 3: | 0 | 0 | **100** | **95** | **95** | **100** |
| position : 135 | Antisense UGAAGUGACCUCAGAGACU dTdT | 3: | | | | | | |
| EGFP | Sense GCAAGCUGACCCUGAAGUUCAU dTdT | 1: | 0 | 0 | 0 | 0 | 0 | 0 |
| | Antisense GAACUUCAGGGUCAGCUUGCCG dTdT | 1 : | | | | | | |

## Claims

1. A method for investigating an epithelial cancer, the method comprising detecting and/or measuring the expression product of the CGB2 and/or CGB1 gene(s), wherein the expression product of the CGB2 and/or CGB1 gene is the 230bp splice variant of the CGB2 and/or CGB1 gene(s) of SEQ ID NO: 1, or the peptide, pre-protein or protein encoded by SEQ ID NO: 1, optionally further comprising detecting and/or measuring the expression of any one or more of the genes CGB3 CGB5, CGB7, and/or CGB8.

2. A method according to claim 1, wherein detecting and/or measuring the expression product of the CGB2 and/or CGB1 gene(s) comprises detecting the levels of mRNA, cDNA, peptide or protein.

3. A method according claim 1 or claim 2, wherein detecting and/or measuring the expression product of the CGB2 and/or CGB1 gene(s) comprises detecting the levels of mRNA or cDNA, preferably wherein the method utilises a primer which hybridises to a sequence within Exon 1 of the CGB2 and/or CGB1 gene and/or a reverse primer which hybridises to a sequence after the initiation site in Exon 2 of the CGB2 and/or CGB1 gene,
optionally wherein the forward primer hybridises to a sequence at the Exon1/Exon2 boundary of the CGB2 and/or CGB1 gene;
or optionally wherein the forward primer is 5'- CGTCCAACACCCCTCACTCC-3' (SEQ ID No: 4.) and/or the reverse primer is 5'- 5 GGCAGCCCTCCTTCTCCAC-3' (SEQ ID. NO:5).

4. A method according to claim 3 further comprising utilising the forward primer 5'- CAGCACCTTTCTCGGGTCAC-3' (SEQ ID No: 6.) and/or the reverse primer 5'-CAGGGAGTAGGGTGTAGGAAGG-3' (SEQ ID. NO:7).

5. A method according to claim 3 or claim 4, wherein the method employs nucleic acid hybridisation for the amplification of CGB genes.

6. A method according to any one of claims 3 to 5 utilising a nucleic acid probe capable of hybridising to the nucleic acid expression product of the CGB2 and/or CGB1 gene(s), optionally wherein the nucleic acid probe forms part of an array.

7. A method according to claim 1 wherein detecting and/or measuring the expression product of the CGB2 and/or CGB1 gene(s) comprises detecting the levels of the peptide, protein or pre-protein, preferably wherein the peptide, protein or pre-protein comprises SEQ ID No.2.

8. A method any of the preceding claims, wherein said method is carried out on a sample obtained from a subject, optionally wherein said sample is selected from a blood, urine, cerebrospinal fluid, sputum, bronchial lavage, and saliva or tissue sample, preferably wherein said sample comprises circulating tumour cells and/or stem cells isolated from the blood of a subject.

9. A kit of parts for detecting and/or measuring the expression of the CGB2 and/or CGB1 gene(s) comprising an agent for detecting and/or measuring the expression product of the CGB2 and/or CGB1 gene(s), wherein the expression product of the CGB2 and/or CGB1 gene is the 230bp splice variant of the CGB2 and/or CGB1 gene(s) of SEQ ID NO: 1, and wherein the agent for detecting and/or measuring the expression of the CGB2 and/or CGB1 gene(s) is selected from any one or more of:
(a) a set of primers capable of hybridising to the 230bp splice variant transcript, wherein the forward primer is capable of hybridising to a sequence at the Exon1/Exon2 boundary; and
(b) a probe capable of selectively hybridizing to the 230bp splice variant transcript.

10. The kit of claim 9 further comprising primers or hybridisation probes capable of hybridising with a housekeeping gene, used as a control.

11. An agent capable of reducing the level of secreted hCGβ for use in medicine, wherein the agent capable of reducing the level of secreted hCGβ is an agent that reduces the expression of the 230bp splice variant of the CGB2 and/or CGB1 gene(s) of SEQ ID NO1, preferably wherein the agent is siRNA molecules which specifically bind to the sequence of the CGB genes.

12. An agent capable of reducing the level of secreted hCGβ for use in a method of treating an hCGβ secreting cancerous condition, wherein said method comprises administering to patient in need thereof, a pharmaceutically acceptable amount of the agent, wherein the agent is an agent that reduces the expression of 230bp splice variant of the CGB2 and/or CGB1 gene(s) of SEQ ID NO: 1, preferably wherein the agent is siRNA molecules which specifically bind to the sequence of the CGB genes.

13. The agent capable of reducing the level of secreted hCGβ for use of claim 12, wherein the cancerous condition is an epithelial cancer.

14. A pharmaceutical composition comprising an agent capable of reducing the level of secreted hCGβ and a pharmaceutically acceptable carrier, diluent or excipient, wherein the agent capable of reducing the level of secreted hCGβ is an agent that reduces the expression of the 230bp splice variant of the CGB2 and/or CGB1 gene(s) of SEQ ID NO: 1, preferably wherein the agent is siRNA molecules which specifically bind to the sequence of the CGB genes.

15. An isolated expression product of CGB1 or CGB2, wherein the expression product is the 230bp splice variant transcript, wherein the expression product comprises a nucleic acid selected from:
(a) A nucleic acid of SEQ. ID NO: 1;
(b) A nucleic acid sequence encoding the polypeptide sequence of SEQ. ID No: 2 or SEQ ID No: 15; and
(c) A sequence having at least 90% identity to the sequence of (a) or (b).

16. An isolated expression product of CGB1 or CGB2, wherein the expression product is an amino acid sequence, or is a polypeptide of SEQ ID NO: 2 or SEQ ID NO: 15 or a polypeptide having at least 90% identity thereto.

## Patentansprüche

1. Verfahren zum Untersuchen von Epithelkrebs, wobei das Verfahren das Erkennen und/ oder Messen des Expressionsprodukts des (der) CGB2 und/ oder CGB1 Gens (Gene) umfasst, wobei das Expressionsprodukt des CGB2 und/ oder CGB1 Gens die 230bp Spleissvariante des (der) CGB2 und/ oder CGB1 Gens (Gene) von SEQ ID NO:1 oder das durch SEQ ID NO:1 codierte Peptid, Präprotein oder Protein ist, optional weiter das Erkennen und/ oder Messen der Expression von einem oder mehreren der Gene CGB3, CGB5, CGB7 und/ oder CGB8 umfassend.

2. Verfahren nach Anspruch 1, wobei das Erkennen und/ oder Messen des Expressionsprodukts des (der) CGB2 und/ oder CGB1 Gens (Gene) das Erkennen der Niveaus von mRNA, cDNA, Peptid oder Protein umfasst.

3. Verfahren nach Anspruch 1 oder Anspruch 2, wobei das Erkennen und/ oder Messen des Expressionsprodukts des (der) CGB2 und/ oder CGB1 Gens (Gene) das Erkennen der Niveaus von mRNA, cDNA umfasst, wobei das Verfahren vorzugsweise einen Primer nutzt, der auf eine Sequenz innerhalb von Exon 1 des CGB2 und/ oder CGB1 Gens und/ oder einen Rückwärtsprimer, der mit einer Sequenz nach der Einleitungsstelle in Exon 2 des CGB2 und/ oder CGB1 Gens hybridisiert,
wobei optional der Vorwärtsprimer mit einer Sequenz an der Exon1/Exon2 Grenze des CGB2 und/ oder CGB1 Gens hybridisiert;
oder wobei optional der Vorwärtsprimer 5'-CGTCCAACACCCCTCACTCC-3' (SEQ ID NO:4) und/ oder der Rückwärtsprimer 5'-5GGCAGCCCTCCTTCTCCAC-3' (SEQ ID NO:5) ist.

4. Verfahren nach Anspruch 3, weiter umfassend das Verwenden des Vorwärtsprimers 5'-CAGCACCTTTCTCGGGTCAC-3' (SEQ ID NO:6) und/ oder des Rückwärtsprimers 5'CAGGGAGTAGGGTGTAGGAAGG-3' (SEQ ID NO:7).

5. Verfahren nach Anspruch 3 oder Anspruch 4, wobei das Verfahren Nukleinsäurehybridisierung zur Verstärkung von CGB-Genen einsetzt.

6. Verfahren nach einem der Ansprüche 3 bis 5, unter Verwendung eines Nukleinsäuretesters, der in der Lage ist, das Nukleinsäure-Expressionsprodukt des (der) CGB2 und/ oder CGB1 Gens (Gene) zu hybridisieren, wobei der Nukleinsäuretester optional einen Teil eines Arrays bildet.

7. Verfahren nach Anspruch 1, wobei das Erkennen und/ oder Messen des Expressionsprodukts des (der) CGB2 und/ oder CGB1 Gens (Gene) das Erkennen der Niveaus des Peptids, Proteins oder Präproteins umfasst, wobei das Peptid, Protein oder Präprotein vorzugsweise SEQ ID No. 2 umfasst.

8. Verfahren nach einem der vorstehenden Ansprüche, wobei das Verfahren an einer Probe ausgeführt wird, die von einem Patienten erhalten wird, wobei die Probe optional aus Blut, Urin, Rückenmarksflüssigkeit, Sputum, Bronchiallavage, und Speichel oder einer Gewebeprobe ausgewählt wird, wobei die Probe vorzugsweise zirkulierende Tumorzellen und/ oder Stammzellen umfasst, die aus dem Blut eines Patienten isoliert sind.

9. Baukastensystem zum Erkennen und/ oder Messen der Expression des (der) CGB2 und/ oder CGB1 Gens (Gene), umfassend ein Mittel zum Erkennen und/ oder Messen des Expressionsprodukts des (der) CGB2 und/ oder CGB1 Gens (Gene), wobei das Expressionsprodukt des (der) CGB2 und/ oder CGB1 Gens (Gene) die 230bp Spleissvariante des (der) CGB2 und/ oder CGB1 Gens (Gene) von SEQ ID NO:1 ist, und wobei das Mittel zum Erkennen und/ oder Messen der Expression des (der) CGB2 und/ oder CGB1 Gens (Gene) aus einem beliebigen oder mehr ausgewählt ist von:
(a) einer Reihe von Primern, die in der Lage sind, auf die 230bp Spleissvariantenabschrift zu hybridisieren, wobei der Vorwärtsprimer in der Lage ist, mit einer Sequenz an der Exon1/Exon2 Grenze zu hybridisieren; und
(b) einen Tester, der in der Lage ist, selektiv mit der 230bp Spleissvariantenabschrift zu hybridisieren.

10. Baukasten nach Anspruch 9, weiter Primer oder Hybridisierungstester umfassend, die in der Lage sind, mit einem Haushaltsgen, das als Kontrolle verwendet wird, zu hybridisieren.

11. Mittel, das in der Lage ist, das Niveau des abgesonderten hCGß zu senken, zur Verwendung in der Medizin, wobei das Mittel, das in der Lage ist, das abgesonderte hCGß zu senken, ein Mittel ist, das die Expression der 230bp Spleissvariante des (der) CGB2 und/ oder CGB1 Gens (Gene) aus SEQ ID NO 1 zu senken, wobei das Mittel vorzugsweise siRNA Moleküle sind, die sich speziell an die Sequenz der CGB Gene binden.

12. Mittel, das in der Lage ist, das Niveau des abgesonderten hCGß zu senken, zur Verwendung bei einem Verfahren zur Behandlung eines hCGß absondernden Krebszustandes, wobei das Verfahren das Verabreichen an den davon betroffenen Patienten, einer pharmazeutisch verträglichen Menge des Mittels umfasst, wobei das Mittel ein Mittel ist, das die Expression der 230bp Spleissvariante des (der) CGB2 und/ oder CGB1 Gens (Gene) aus SEQ ID NO 1 senkt, wobei das Mittel vorzugsweise siRNA Moleküle sind, die sich speziell an die Sequenz der CGB Gene binden.

13. Mittel, das in der Lage ist, das Niveau des abgesonderten hCGß zu senken, zur Verwendung nach Anspruch 12, wobei der Krebszustand ein Epithelkrebs ist.

14. Pharmazeutische Zusammensetzung, ein Mittel, das in der Lage ist, das Niveau des abgesonderten hCGß zu senken, und einen pharmazeutisch verträglichen Trägerstoff, Verdünner oder Hilfsstoff umfassend, und wobei das Mittel, das in der Lage ist, das abgesonderte hCGß zu senken, ein Mittel ist, das die Expression der 230bp Spleissvariante des (der) CGB2 und/ oder CGB1 Gens (Gene) aus SEQ ID NO 1 senkt, wobei das Mittel vorzugsweise siRNA Moleküle sind, die sich speziell an die Sequenz der CGB Gene binden.

15. Isoliertes Expressionsprodukt von CGB1 oder CGB2, wobei das Expressionsprodukt die 230bp Spleissvariantenabschrift ist, wobei das Expressionsprodukt eine Nukleinsäure ist, ausgewählt aus:
(a) einer Nukleinsäure der SEQ ID NO: 1;
(b) einer Nukleinsäuresequenz, die die Polypeptidsequenz von SEQ ID NO: 2 oder SEQ ID NO: 15 codiert; und
(c) einer Sequenz, die mindestens 90% Identität mit der Sequenz aus (a) oder (b) aufweist.

16. Isoliertes Expressionsprodukt von CGB1 oder CGB2, wobei das Expressionsprodukt eine Aminosäuresequenz ist oder ein Polypeptid aus SEQ ID NO: 2 oder SEQ ID NO: 15 oder ein Polypeptid ist, das mindestens 90% Identität damit aufweist.

## Revendications

1. Procédé pour examiner un cancer épithélial, le procédé comprenant la détection et/ou la mesure du produit d'expression du gène CGB2 et/ou du gène CGB1, dans lequel le produit d'expression du gène CGB2 et/ou du gène CGB1 est le variant d'épissage de 230 pb du gène CGB2 et/ou du gène CGB1 de SEQ ID NO : 1, ou le peptide, la préprotéine ou la protéine codé par SEQ ID NO : 1, facultativement comprenant en outre la détection et/ou la mesure de l'expression de l'un quelconque ou plusieurs des gènes CGB3 CGB5, CGB7 et/ou CGB8.

2. Procédé selon la revendication 1, dans lequel la détection et/ou la mesure du produit d'expression du gène CGB2 et/ou du gène CGB1 comprennent la détection des taux d'un ARNm, d'un ADNc, d'un peptide ou d'une protéine.

3. Procédé selon la revendication 1 ou la revendication 2, dans lequel la détection et/ou la mesure du produit d'expression du gène CGB2 et/ou du gène CGB1 comprennent la détection des taux d'un ARNm ou d'un ADNc, de préférence dans lequel le procédé utilise une amorce qui s'hybride à une séquence à l'intérieur de l'exon 1 du gène CGB2 et/ou du gène CGB1 et/ou une amorce antisens qui s'hybride à une séquence après le site d'initiation dans l'exon 2 du gène CGB2 et/ou du gène CGB1,
facultativement dans lequel l'amorce sens s'hybride à une séquence à la limite exon1/exon2 du gène CGB2 et/ou du gène CGB1 ;
ou facultativement dans lequel l'amorce sens est 5'-CGTCCAACACCCCTCACTCC-3' (SEQ ID NO : 4) et/ou l'amorce antisens est 5'- 5 GGCAGCCCTCCTTCTCCAC-3' (SEQ ID NO : 5).

4. Procédé selon la revendication 3, comprenant en outre l'utilisation de l'amorce sens 5'-CAGCACCTTTCTCGGGTCAC-3' (SEQ ID No : 6.) et/ou de l'amorce antisens 5'-CAGGGAGTAGGGTGTAGGAAGG-3' (SEQ ID. NO : 7).

5. Procédé selon la revendication 3 ou la revendication 4, dans lequel le procédé utilise une hybridation d'acide nucléique pour l'amplification des gènes CGB.

6. Procédé selon l'une quelconque des revendications 3 à 5 utilisant une sonde d'acide nucléique capable de s'hybrider au produit d'expression d'acide nucléique du gène CGB2 et/ou du gène CGB1, facultativement dans lequel la sonde d'acide nucléique forme une partie d'un réseau.

7. Procédé selon la revendication 1, dans lequel la détection et/ou la mesure du produit d'expression du gène CGB2 et/ou du gène CGB1 comprennent la détection des taux du peptide, de la protéine ou de la préprotéine, de préférence dans lequel le peptide, la protéine ou la préprotéine comprend SEQ ID No. 2.

8. Procédé selon l'une quelconque des revendications précédentes, dans lequel ledit procédé est mis en œuvre sur un échantillon obtenu à partir d'un sujet, facultativement dans lequel ledit échantillon est sélectionné parmi un échantillon de sang, d'urine, de liquide céphalo-rachidien, d'expectoration, de lavage bronchique et de salive ou de tissu, de préférence dans lequel ledit échantillon comprend des cellules tumorales circulantes et/ou des cellules souches isolées à partir du sang d'un sujet.

9. Kit de parties pour détecter et/ou mesurer l'expression du gène CGB2 et/ou du gène CGB1 comprenant un agent pour détecter et/ou mesurer le produit d'expression du gène CGB2 et/ou du gène CGB1, dans lequel le produit d'expression du gène CGB2 et/ou du gène CGB1 est le variant d'épissage de 230 pb du gène CGB2 et/ou du gène CGB1 de SEQ ID NO : 1, et dans lequel l'agent pour détecter et/ou mesurer l'expression du gène CGB2 et/ou du gène CGB1 est sélectionné parmi l'un quelconque ou plusieurs parmi :
(a) un ensemble d'amorces capables de s'hybrider au produit de transcription du variant d'épissage de 230 pb, dans lequel l'amorce sens est capable de s'hybrider à une séquence à la limite exon1/exon2 ; et
(b) une sonde capable de s'hybrider sélectivement au produit de transcription du variant d'épissage de 230 pb.

10. Kit selon la revendication 9, comprenant en outre des amorces ou des sondes d'hybridation capables de s'hybrider à un gène domestique, utilisé comme témoin.

11. Agent capable de réduire le taux de hCGβ sécrété pour une utilisation en médecine, dans lequel l'agent capable de réduire le taux de hCGβ sécrété est un agent qui réduit l'expression du variant d'épissage de 230 pb du gène CGB2 et/ou du gène CGB1 de SEQ ID NO : 1, de préférence dans lequel l'agent est des molécules d'ARNsi qui se lient spécifiquement à la séquence des gènes CGB.

12. Agent capable de réduire le taux de hCGβ sécrété pour une utilisation dans un procédé de traitement d'une affection cancéreuse sécrétant hCGβ, dans lequel ledit procédé comprend l'administration à un patient en ayant besoin, d'une quantité pharmaceutiquement acceptable de l'agent, dans lequel l'agent est un agent qui réduit l'expression du variant d'épissage de 230 pb du gène CGB2 et/ou du gène CGB1 de SEQ ID NO : 1, de préférence dans lequel l'agent est des molécules d'ARNsi qui se lient spécifiquement à la séquence des gènes CGB.

13. Agent capable de réduire le taux de hCGβ sécrété pour une utilisation selon la revendication 12, dans lequel l'affection cancéreuse est un cancer épithélial.

14. Composition pharmaceutique comprenant un agent capable de réduire le taux de hCGβ sécrété et un support, diluant ou excipient pharmaceutiquement acceptable, dans lequel l'agent capable de réduire le taux de hCGβ sécrété est un agent qui réduit l'expression du variant d'épissage de 230 pb du gène CGB2 et/ou du gène CGB1 de SEQ ID NO : 1, de préférence dans lequel l'agent est des molécules d'ARNsi qui se lient spécifiquement à la séquence des gènes CGB.

15. Produit d'expression isolé de CGB1 ou CGB2, dans lequel le produit d'expression est le produit de transcription du variant d'épissage de 230 pb, dans lequel le produit d'expression comprend un acide nucléique sélectionné parmi :
(a) un acide nucléique de SEQ. ID NO : 1 ;
(b) une séquence d'acide nucléique codant pour la séquence polypeptidique de SEQ. ID No : 2 ou de SEQ ID No : 15 ; et
(c) une séquence présentant une identité d'au moins 90 % avec la séquence de (a) ou (b).

16. Produit d'expression isolé de CGB1 ou CGB2, dans lequel le produit d'expression est une séquence d'acides aminés, ou est un polypeptide de SEQ ID NO : 2 ou de SEQ ID NO : 15 ou un polypeptide présentant une identité d'au moins 90 % avec celles-ci.
